(19) Europäisches Patentamt
European Patent Office
Office européen des brevets

(11) **EP 4 394 041 A1**

(12) **EUROPEAN PATENT APPLICATION**
published in accordance with Art. 153(4) EPC

(43) Date of publication:
**03.07.2024 Bulletin 2024/27**

(21) Application number: **22860104.3**

(22) Date of filing: **15.07.2022**

(51) International Patent Classification (IPC):
*C12N 15/85* (2006.01)    *C07K 19/00* (2006.01)
*C12N 15/62* (2006.01)    *A61K 38/20* (2006.01)
*A61K 47/64* (2017.01)    *A61P 1/00* (2006.01)

(86) International application number:
**PCT/CN2022/106105**

(87) International publication number:
**WO 2023/024758 (02.03.2023 Gazette 2023/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.08.2021 CN 202110982310**

(71) Applicant: **Beijing Vdjbio Co., Ltd.
Beijing 100085 (CN)**

(72) Inventors:
• **ZHANG, Xiaorui
Beijing 100085 (CN)**
• **ZHANG, Jun
Beijing 100085 (CN)**
• **ZHAO, Tingting
Beijing 100085 (CN)**

• **CHENG, Jianwei
Beijing 100085 (CN)**
• **MA, Rong
Beijing 100085 (CN)**
• **SUN, Yiping
Beijing 100085 (CN)**
• **TIAN, Xinsheng
Beijing 100085 (CN)**
• **LI, Ziqiang
Beijing 100085 (CN)**

(74) Representative: **Dolphin, Kirsty Mairi et al
Venner Shipley LLP
200 Aldersgate
London EC1A 4HD (GB)**

Remarks:
The complete document including Reference
Table(s) and the Sequence Listing(s) can be
downloaded from the EPO website

(54) **FUSION PROTEIN OF INTERLEUKIN 2 AND APPLICATION THEREOF IN IBD**

(57)     Disclosed is a fusion protein of interleukin 2 and an application thereof in the treatment or prevention of inflammatory bowel disease, characterized in that the fusion protein comprises: human interleukin 2 or a variant thereof, and human serum albumin or a variant thereof.

EP 4 394 041 A1

**Description**

TECHNICAL FIELD

The present disclosure belongs to the technical field of biopharmaceutical. In particular, the present disclosure relates to a fusion protein of interleukin-2 and its use in the treatment or prevention of Inflammatory bowel disease (IBD).

BACKGROUND

Inflammatory bowel disease (IBD) is a group of chronic, non-specific intestinal inflammation with unknown etiology. IBD has clinical symptoms such as abdominal pain, diarrhea and mucosanguineous feces. However, the etiopathogenesis of IBD remains largely unknown. Existing studies show that IBD is closely related to many factors, for example hosts' genetic susceptibility, intestinal flora disorder, intestinal mucosal barrier injury, intestinal mucosal immune abnormalities, environment and mental state. IBD has characteristics such as incurableness, lifelong recrudescence and disability, which brings great physical pain and mental burden to patients.

The goal of the treatment of IBD is to induce and maintain clinical remission and mucosal healing, to prevent the complications, to improve the quality of life of patients, and to strengthen the long-term management of patients. At present, for internal-medicine department, most of the drugs for active IBD are still used for blocking or interrupting inflammatory cascades, which can only partially relieve the symptoms of patients, but cannot completely cure the disease. At present, the treatment of IBD includes non-targeted therapies, targeted biotherapies, induction therapies and other novel therapies. Such treatments are described in detail below.

The non-targeted therapies include aminosalicylates, glucocorticoids, and immunomodulatory agents. Among them, aminosalicylates are mainly effective in mild and moderate ulcerative colitis (UC); glucocorticoids and immunosuppressive agents have extensive side effects.

The targeted biotherapies mainly uses biological agents for treatment, which include the following mechanism of action: 1. targeted blocking of the biological effects of various pro-inflammatory cytokines involved in the intestinal mucosal inflammation, such as anti-TNF-$\alpha$ antibodies (Infliximab, Adalimumab, Golimumab, Cetuzumab) and anti-IL12/23P40 antibodies (Ustekinumab); 2. inhibition of the migration of activated white blood cells to the intestinal mucosa, such as anti-integrin antibodies (Vedolizumab, Natalizumab). The targeted biotherapies can, in different degree, improve the clinical remission rate of IBD patients, reduce the recurrence rate and surgical rate, thereby providing more treatment options for IBD patients. However, up to 30% of the patients are primarily unresponsive to, and up to 50% are secondarily unresponsive to the biological agents.

The induction therapies mainly uses oral small-molecules, such as selective JAK inhibitors (Tofacitinib) and sphingosin-1-phosphate (S1P) receptor modulators (Ozanimod). Such small molecule drugs can also improve the clinical remission rate of IBD patients, reduce the recurrence rate and surgical rate, thereby providing more treatment options for IBD patients. The small molecule drugs overcome the compliance problem of the patients caused by the immunogenicity and parenteral administration route of the biological agents. However, the small molecule drugs may induce drug interaction and produce toxicity during the treatment of IBD, resulting in treatment failure. There is risk of serious infection, malignant tumor and thrombosis for the JAK inhibitors on the markets, and is risk of cardiovascular disorders for Ozanimod.

The other novel therapies include stem cell regeneration, barrier-repairing agents, fecal flora transplantation and the like.

Regulatory T ($T_{reg}$) cells are a subpopulation of T cells that expresses Foxp3, CD25 and CD4. $T_{reg}$ cells can inhibit the activation and proliferation of potential autoreactive T cells in normal bodies in a way of active regulation, and involve in the maintenance of immunologic tolerance of the bodies. It has been confirmed that $T_{reg}$ cells play a critical role in the pathogenesis of IBD, as well as in other autoimmune diseases. In normal intestinal mucosa, the effector T ($T_{eff}$) cells and $T_{reg}$ cells are in a state of dynamic balance to maintain the stability of the intestinal environment. Once the balance is broken due to, for example excessive inflammatory effect or reduced regulatory effect, it may lead to the occurrence of IBD.

At present, it has become research hotspots to reduce intestinal inflammation in IBD by increasing the number and / or function of $T_{reg}$ cells through various ways. It generally includes: micropopulation which is capable of promoting the differentiation of $T_{reg}$ cells; low-dose interleukin 2 (IL-2) (since $T_{reg}$ cells inherently express high-affinity IL-2 receptors, low-dose IL-2 can selectively increase the number of $T_{reg}$ cells, but not $T_{eff}$ cells); IL-2-anti-IL-2 antibody complexes which have *in vivo* stability; engineered IL-2 variants (also called mutant proteins, which can selectively bind the high-affinity IL-2 receptor); adoptive $T_{reg}$ cell therapies; and the like.

Although the treatment of IBD has been improving, the long-term colectomy rate in UC patients has not been decreased in the past decade. In other words, there are still large and unmet clinical needs for IBD disease. There is an urgent need for new therapeutic drugs or approaches to improve the quality of life of IBD patients.

EP 4 394 041 A1

SUMMARY OF THE INVENTION

The purpose of the disclosure is to provide a fusion protein of interleukin 2 and its use in the treatment or prevention of inflammatory bowel disease (IBD), with prolonged administration interval, alleviated pain of patients, reduced cost, and improved compliance and life quality of patients.

According to the first aspect of the present disclosure, a fusion protein of interleukin-2 is provided. The fusion protein comprises a human interleukin 2 or its variant, and a human serum albumin or its variant.

The human interleukin 2 or its variants may include: an amino acid sequence as shown in SEQ ID NO:1; or, an amino acid sequence having at least 90% sequence identity with the amino acid sequence as shown in SEQ ID NO: 1.

The human serum albumin or its variants may include: an amino acid sequence as shown in SEQ ID NO:2; or, an amino acid sequence having at least 90% sequence identity with the amino acid sequence as shown in SEQ ID NO:2.

In the present disclosure, the amino acid sequence as shown in SEQ ID NO:1 is human interleukin 2 with 133 amino acid residues, and the amino acid sequence as shown in SEQ ID NO:2 is human serum albumin with 585 amino acid residues.

In some embodiments, the fusion protein of the interleukin 2 may include the amino acid sequence as shown in SEQ ID NO: 1 and the amino acid sequence as shown in SEQ ID NO:2.

In some embodiments, the amino acid at position 125 of the amino acid sequence as shown in the SEQ ID NO: 1 is not cysteine. The human interleukin 2 (IL-2) has an intrachain disulfide bond. When the amino acid at position 125 of the mature protein is cysteine, it may tend to form mismatched disulfide bonds with the other two cysteines, resulting in the loss of the activity of IL-2.

In some embodiments, the amino acid at position 125 of the amino acid sequence as shown in SEQ ID NO:1 may be replaced with serine or alanine. The mismatched disulfide bonds can be avoided through the mutation of the amino acid residue at position 125 of human interleukin 2 to serine or alanine, allowing the human interleukin 2 to remain active.

In some embodiments, the human interleukin 2 or its variant may be linked to the human serum albumin or its variant directly or via a linking peptide.

In some embodiments, the linking peptide is provided between the human interleukin 2 or its variant and the human serum albumin or its variant. There may be a large interval between the two units contained in the fusion protein of interleukin 2, so that the human interleukin 2 has the maximum probability of binding to the interleukin 2 receptor.

In some embodiments, the linking peptide may have a general formula of $(G_nS)_m$, in which n or m is an integer selected from 1 to 10.

In some embodiments, the linking peptide may have a general formula of $(G_nS)_m$, in which n is an integer selected from 1 to 4 and m is an integer selected from 0 to 3.

In some embodiments, the linking peptide may have a general formula of $(G_nS)_m$, in which n is an integer selected from 1-4 and m is an integer selected from 1-3.

In some embodiments, the linking peptide may have an amino acid sequence of GGGGSGGGGS.

In some embodiments, the linking peptide may have an amino acid sequence of GGGGS.

In some embodiments, the fusion protein may have a signal peptide at the N-terminal thereof. The addition of the signal peptide to the N-terminal of the fusion protein can further increase the expression level of the fusion protein.

In some embodiments, the signal peptide may be derived from the signal peptide of CD33 for secretion.

In some embodiments, the nucleic acid sequence encoding the signal peptide may be shown as SEQ ID NO:5.

In some embodiments, the fusion protein may have an amino acid sequence as shown in SEQ ID NO:4.

In some embodiments, the fusion proteins may comprise: from N-terminal, human interleukin 2-linking peptide-human serum albumin, or human serum albumin -linking peptide-human interleukin 2.

In some embodiments, the amino acid sequence of SEQ ID NO:4 may be a protein having 728 amino acid residues, of which amino acids 1 to 133 are the human interleukin 2, amino acids 134 to 143 are the linking peptide GGGGSGGGGS, and amino acids 144 to 728 are the human serum albumin.

According to the second aspect of the present disclosure, an isolated nucleic acid molecule is provided. The nucleic acid molecule may encode the fusion protein provided in the first aspect of the present disclosure.

In some embodiments, the fusion protein may be encoded by the nucleotide sequence as shown in SEQ ID NO:3.

In the present disclosure, the codon optimization may be performed on the nucleotide sequences of the human interleukin 2 and human serum albumin disclosed on Genbank, according to the mammalian codon preference. The optimized nucleotide sequence of the fusion protein may be inserted in an expression vector, which is then transfected into a host cell for expression. The target protein may be purified, i.e., the fusion protein of interleukin 2.

The expression system of the fusion protein having the interleukin-2 and human serum albumin, can be transfected (for example, electrotransfer) into CHO cells with a plasmid carrying the fusion gene encoding the interleukin-2 and human serum albumin. A CHO monoclonal cell line which can stably and efficiently express the human recombinant protein, is obtained. Further, the monoclonal cell line of the present disclosure can express and secrete the fusion protein

3

having the interleukin 2 and human serum albumin (i.e., the fusion protein of interleukin 2). It can greatly prolong the plasma half-life of human interleukin 2, and thus can be used for preparing the medicament associated with the expression of human interleukin 2.

In some embodiments, the expression vector may be selected from pEE14.4, pcDNA3.1 and pEE6.4, and preferably be pEE14.4.

According to the third aspect of the present disclosure, an expression system is provided. The expression system may comprise a CHO cell. The CHO cell may contain the nucleic acid molecule provided in the second aspect of the present disclosure.

The expression system may express the fusion protein provided in the first aspect of the present disclosure.

The present disclosure unexpectedly finds that the CHO cell, especially the CHO-K1 cell line, has a high expression level of the fusion protein, up to 4g /L. The expressed fusion protein of interleukin 2 is highly active, and can strongly promote, *in vitro,* the proliferation of specific cells such as NK, T and/or $T_{reg}$ cells.

In some embodiments, the expression system may be a CHO-K1 cell, which is deposited in China General Microbiological Culture Collection Center on May 26, 2022, under CGMCC No. 45173.

According to the fourth aspect of the present disclosure, a pharmaceutical composition is provided. The pharmaceutical composition may comprise the fusion protein provided in the first aspect of the present disclosure, and a pharmaceutically acceptable carrier.

In some embodiments, the pharmaceutical composition may be in a dosage form of injection, tablet, or capsule.

In some embodiments, the pharmaceutical composition may be in a dosage form of solution for injection or lyophilized powder for injection.

In some embodiments, the pharmaceutical composition may include a pharmaceutically acceptable carrier.

In some embodiments, the carrier may be selected from excipient, diluent, filler, binder, wetting agent, disintegrant, absorption enhancer, surfactant, adsorption support, and/or stabilizer.

According to the fifth aspect of the present disclosure, provided is a method for treating or preventing inflammatory bowel disease (IBD). The method may comprise administrating to a subject of an effective amount of the fusion protein provided by the first aspect of the present disclosure, or the pharmaceutical composition provided by the fourth aspect of the present disclosure.

In some embodiments, the IBD may comprise ulcerative colitis, Crohn's disease and indeterminate colitis.

In some embodiments, the fusion protein or the pharmaceutical composition may be administered by injection.

In some embodiments, the fusion protein or the pharmaceutical composition may be administered by subcutaneous or intravenous infusion.

In some embodiments, the fusion protein or the pharmaceutical composition may be administered by subcutaneous injection.

In some embodiments, the fusion protein may be administered at $3\times10^4$IU to $1\times10^6$IU each dose.

In some embodiments, the pharmaceutical composition may be administered at $3\times10^4$IU to $1\times10^6$IU each dose, as measured by the fusion protein therein.

In some embodiments, the fusion protein may be administered every 7-28 days.

In some embodiments, the fusion protein may be administered every 14-28 days.

In some embodiments, the pharmaceutical composition may be administered every 7-28 days.

In some embodiments, the pharmaceutical composition may be administered every 14-28 days.

According to the sixth aspect of the present disclosure, provided is use of the fusion protein provided in the first aspect of the present disclosure in the treatment or prevention of inflammatory bowel disease (IBD).

According to the seventh aspect of the present disclosure, provided is use of the pharmaceutical composition provided in the fourth aspect of the present disclosure in the treatment or prevention of inflammatory bowel disease (IBD).

According to the eighth aspect of the present disclosure, provided is use of the fusion protein provided in the first aspect of the present disclosure in the preparation of a medicament for treating or preventing inflammatory bowel disease (IBD).

According to the ninth aspect of the present disclosure, provided is use of the pharmaceutical composition provided in the fourth aspect of the present disclosure in the preparation of a medicament for treating or preventing inflammatory bowel disease (IBD).

The fusion protein of interleukin-2 of the present disclosure has higher biological activity and longer plasma half life.

The fusion protein of interleukin 2 of the present disclosure is capable of treating or preventing IBD.

The fusion protein of interleukin 2 of the present disclosure is capable of increasing the therapeutic effect for IBD patients, further improving diarrhea, colopathy and the like.

The fusion protein of interleukin 2 provided by the present disclosure has slowed-down hydrolysis in patients, significantly prolonged interval time of the administration, enhanced therapeutic effect, reduced total administration dosage. Correspondingly, the fusion protein of the present disclosure can relieve the patients' pain, reduce treatment cost, reduce the occurrence of adverse reactions, thereby improving the compliance and life quality of the patients. Therefore, the present disclosure brings hope to the recovery of IBD patients.

DESCRIPTION OF THE DRAWINGS

FIG. 1 shows the reducing electrophoresis results of the supernatants of IL-2-HSA / CHO-K1 clones cultured in a well plate. 20 $\mu$L of the supernatants were loaded for each lane.

FIG. 2 shows the non-reducing electrophoresis results of the fed-batch culture supernatants on D13 of IL-2-HSA/CHO-K1 clones which were screened, and the protein expression of each cell line. 5 $\mu$L of the supernatants were loaded for each lane. FIG. 2A shows the non-reducing electrophoresis results of the fed-batch culture supernatants of the screened IL-2-HSA / CHO-K1 clones on D13; FIG. 2B shows the protein expression of each cell line.

FIG. 3 shows the non-reducing electrophoresis results of the fed-batch culture supernatants on D13 of IL-2-HSA / CHO-K1 clone # 9 which was subjected for monoclonal screening, and the protein expression of each cell line. 3 $\mu$L of the supernatants were loaded for each lane. FIG. 3A shows the non-reducing electrophoresis results of the fed-batch culture supernatant on D13 of IL-2-HSA / CHO-K1 clone # 9 which was subjected for monoclonal screening; FIG. 3B shows the protein expression of each cell line.

FIG. 4 shows the non-reducing electrophoresis results of the fed-batch culture supernatants on D13 of IL-2-HSA / CHO-K1 clone # 9-6 which was subjected for monoclonal screening, and the protein expression of each cell line. 2 $\mu$L of the supernatants were loaded for each lane. FIG. 4A shows the non-reducing electrophoresis results of the fed-batch culture supernatants on D13 of IL-2-HSA / CHO-K1 clone # 9-6 which was subjected for monoclonal screening; FIG. 4B shows protein expression of each cell line.

FIG. 5 shows the kinetics curve of the cultured IL-2-HSA/CHO-K1 cells. The cells were fed-batch cultured in 125 mL shaker with an initial cell density of $0.3\times10^6$ cells / mL, an initial culture volume of 25 mL and a maximum viable cell density of up to $19.3\times10^6$ cells / mL.

FIG. 6 shows the dynamic curve of the protein expression of the supernatants on D7-D13 of the IL-2-HSA / CHO-K1 cells which were seeded at low-density and cultured.

FIG. 7 shows the non-reducing electrophoresis results of the supernatants of the IL-2-HSA / CHO-K1 cells. 1 $\mu$L of the supernatants were loaded for each lane.

FIG. 8 shows the kinetics curve of the cultured IL-2-HSA/CHO-K1 cells. The cells were fed-batch cultured in a 1L shaker with an initial cell density of $2\times10^6$ cells / mL, an initial culture volume of 300 mL and a maximum viable cell density of up to $16\times10^6$ cells / mL.

FIG. 9 shows the dynamic curve of the protein expression of the supernatants on D0-D10 of the IL-2-HSA / CHO-K1 cells which were seeded at high-density and cultured.

FIG. 10 shows the non-reducing electrophoresis results of the supernatants of the IL-2-HSA / CHO-K1 cells. 5 $\mu$L of the supernatants were loaded for each lane.

FIG. 11 shows the proliferation curve of NK-92 stimulated by IL-2-HSA.

FIG. 12 shows the proliferation curve of CTLL-2 stimulated by IL-2-HSA.

FIG. 13 shows the effect of the IL-2-HAS fusion protein on the survival rate of C57BL/6 mice of colonitis models induced by DSS in the recovery phase ($D_8$-$D_{15}$).

FIG. 14 shows the effect of the IL-2-HAS fusion protein on the body weight of C57BL/6 mice of colonitis models induced by DSS.

FIG. 15 shows the effect of the IL-2-HAS fusion protein on the faecal traits of C57BL/6 mice of colonitis models induced by DSS.

FIG. 16 shows the effect of the IL-2-HAS fusion protein on the hematochezia degree of C57BL/6 mice of colonitis models induced by DSS.

FIG. 17 shows the photographs of the colon samples from C57BL/6 mice euthanized on D8.

FIG. 18 shows the effect of the IL-2-HAS fusion protein on the colon length of C57BL/6 mice of colonitis models induced by DSS.

FIG. 19 shows the effect of the IL-2-HAS fusion protein on the colon weight of C57BL/6 mice of colonitis models induced by DSS.

FIG. 20 shows the effect of the IL-2-HAS fusion protein on the spleen weight and organ coefficients of C57BL/6 mice of colonitis models induced by DSS.

FIG. 21 shows the effect of the IL-2-HAS fusion protein on the spleen lymphocyte subpopulation of C57BL/6 mice of colonitis models induced by DSS.

FIG. 22 shows the representative flow cytometry results ($D_8$) of the spleen $T_{reg}$ (CD4$^+$CD25$^+$Foxp3$^+$) cells from the mice of colonitis models.

FIG. 23 shows the representative flow cytometry results ($D_{15}$) of the spleen $T_{reg}$ (CD4$^+$CD25$^+$Foxp3$^+$) cells from the mice of colonitis models.

FIG. 24 shows the determined binding abilities of the IL-2-HSA fusion protein to IL-2R from different species (i.e., human, dog, rat, mouse).

FIG. 25 shows the effect of the IL-2-HSA fusion protein and rhIL-2 on the *in vitro* proliferation of $T_{reg}$ (CD3$^+$CD4$^+$CD25$^+$CD127$^{low/-}$) subpopulations in human PBMC.

FIG. 26 shows the flow cytometry results of $T_{reg}$ (CD3$^+$CD4$^+$CD25$^+$CD127$^{low/-}$) subpopulations in human PBMC.

**DESCRIPTION OF PARTICULAR EMBODIMENTS OF THE INVENTION**

The invention is further illustrated through the examples below. It should be understood that the examples of the present invention are only intended to illustrate the present invention but not to limit it. Any simple improvement made to the present invention under the concept of the present invention is within the scope of protection claimed by the present invention.

The inventors found the decrease in the level of regulatory T ($T_{reg}$) cells was associated with the increase of severity of the disease of IBD patients, and can be used for predicting the progression of the disease and survival, indicating that they may be a potential therapeutic target. The production, activation, and survival of the $T_{reg}$ cells are completely dependent on cytokine interleukin 2 (IL-2). Based on this, the inventors carried out the following experiments.

Definition

The term "fusion protein" as used herein means a bioactive polypeptide (usually TCR or an antibody) and an effector molecule (usually a protein or peptide sequence) which are covalently linked by any appropriate method such as recombinant, chemical or other methods. If desired, one or more molecules may be fused via linking peptides. Alternatively, the linking peptides may be used to assist in the construction of the fusion molecule. The fusion molecule is particularly preferred to be a fusion protein. In general, the fusion molecule may also include conjugated molecules.
The terms "expression vector" and "expression construct" as used herein can be used interchangeably. When the above isolated nucleic acid molecules are connected to the vector, the nucleic acid sequence can be directly or indirectly connected to the regulatory elements on the vector, as long as these regulatory elements can regulate the translation and expression of the nucleic acid molecules. These regulatory elements can be derived directly from the vector itself, or can be exogenous, i.e., be not derived from the vector itself. That is, the nucleic acid molecule is operationally connected to the regulatory element(s). In this disclosure, "operationally connected" means that a foreign gene is connected to the vector so that the regulatory element(s) in the vector (e.g. transcription- and translation- regulatory sequences) can play the intended functions of regulating the transcription and translation of the foreign gene. Of course, the polynucleotides encoding the heavy and light chains of an antibody can be inserted independently into different vectors, often into the same vector. Common vectors may include, for example, plasmids, bacteriophages, and the like.

Compared to a sequence, a variant having "at least 90% sequence identity" with the sequence as used herein may include an amino acid sequence having at least 90%, at least 91%, at least 92%, at least 92%, at least 93%, at least 94%, at least 95%, at least 96%, at least 97%, at least 98%, at least 99% or 100% sequence identity with the sequence, and having identical or similar function to that of the sequence.

The term "pharmaceutically acceptable carrier" as used herein may include any physiologically compatible solvent, dispersion medium, coating, antibacterial agent, antifungal agent, isotonic agent, delayed absorber, and the like. Particular examples may include one or more selected from, e.g. water, saline, phosphate buffered saline (PBS), glucose, glycerol, ethanol, and their combinations. In many cases, the pharmaceutically acceptable carrier may include isotonic agents, such as sugars, polyols (e.g., mannitol, sorbitol), or sodium chloride. The pharmaceutically acceptable carrier may also include a trace amount of auxiliary substances, such as wetting agents, emulsifiers, preservatives or buffers, to extend the shelf life or potency of the protein.

The term "subject" as used herein refers to a subject who have IBD or are at risk of developing IBD. Subjects can include animals, preferably be mammals, and more preferably be humans.

The term "treatment" as used herein includes playing a role in the specific disease, disorder or condition, reducing the severity of the disease, disorder or condition, or delaying or slowing the progression of the disease, disorder or condition.

The term "prevention" as used herein means the likelihood of at least reducing the risk (or susceptibility) of developing a disease or condition (i.e., stopping at least one clinical symptom of the disease that has not developed in a patient who may be exposed or susceptible to the disease but has not experienced or showed the symptoms of the disease).

The term "effective amount" as used herein means an amount of the therapeutic, prophylactic and/or diagnostic agent which is sufficient for the treatment, relievement, improvement, reduction of the symptoms, prevention, suppression of the progression, reduction of the severity, and/or reduction of the incidence of a disease, condition, and / or symptom, when administered to a subject having or being susceptible to the disease, condition, and / or symptom.

The term "dosing" or "dosage" as used herein means an amount that can relieve or delay the progression of a disease, or degenerative or traumatic conditions. The dosage can be determined depending on the specific disease to be treated and other factors, including age, weight, health status, severity of the disease, administration route, frequency of treatment, and whether other medications are accompanied during treatment.

The term "inflammatory bowel disease" or "IBD" as used herein refers to an idiopathic bowel inflammatory disease involving ileum, rectum, and colon. IBD is clinically manifested as diarrhea, abdominal pain, even hematochezia. The etiology and pathogenesis of this disease have not been fully understood. It is known that the inflammatory responses caused by the abnormalities in the intestinal mucosal immune system play important role in the pathogenesis of IBD. It is believed that IBD may be caused by multifactor interactions, mainly including environmental, genetic, infectious and immunological factors.

The term "ulcerative colitis" as used herein refers to a chronic nonspecific, non-infectious, inflammatory intestinal disease mainly involving colorectal mucosa and submucosa. Ulcerative colitis generally show the following features: continuous and diffuse distribution; continuous mucosal ulcer in rectum and colon; different degrees of expansion initiating from rectum, farthest to cecum.

The term "Crohn's disease" as used herein can involve the whole digestive tract, be discontinuous full-thickness inflammation. Crohn's disease usually occurs in terminal ileum, colon and perianal region. This disease usually includes symptoms such as abdominal pain, diarrhea (potentially bleeding if severely inflamed), fever, and weight loss.

The term "indeterminate colitis" as used herein refers to the cases in which there is difficulty in distinguishing between ulcerative colitis and Crohn's disease of IBD patients. Such type of IBD is currently defined as indeterminate colitis.

The solutions of the present invention will be illustrated in combination with the examples below. Those skilled in the art should understand that the following examples are used only to illustrate the invention and not be deemed as limiting the scope of the invention. In the following, the description of the commonly known techniques is omitted to avoid unnecessarily confusing the concept of this disclosure. Such techniques are described in many publications, such as Molecular Cloning: A Laboratory manual (Fourth Edition) (Cold Spring Harbor Laboratory Press).

The examples, which are not indicated with the specific technology or conditions, are performed through the technology or conditions described in the literatures in the field or according to the product specifications. The reagents or instruments used, which are not indicated with the manufacturers, are conventional products that can be available on the market.

The inventors found the decrease of the regulatory T ($T_{reg}$) cells in IBD patients is associated with the increased severity of the disease, and can predict the progression of the disease and the survival rate. It suggests that they may be a potential therapeutic target. In addition, the production, activation, and survival of the $T_{reg}$ cells are completely dependent on cytokine interleukin 2 (IL-2). Based on this, the inventors carried out the following experiments.

Illustrations of the terms and expressions involved in the examples are given below.

IL-2-HSA fusion protein is an abbreviated form of the fusion protein of interleukin 2.

IL-2 refers to human interleukin 2.

HSA refers to human serum albumin.

$^{125}$Ala IL-2 refers to a mutant of interleukin-2 having an amino acid sequence in which the amino acid at position 125 is alanine. See the amino acid sequence shown by SEQ ID NO:1 for details.

$D_0$, $D_2$, $D_4$, $D_8$, $D_{15}$ represent Day 0, Day 2, Day 4, Day 8, and Day 15, respectively.

DSS stands for dextran sodium sulfate.

DSS-period represents the period of drinking water supplemented with dextran sodium sulfate.

s.c. indicates subcutaneous injection.

The trade name of commercially available short-acting IL-2 is Xinjier and is manufactured by Beijing SL Pharm Co., LTD.

## Examples

### Example 1: Construct a stably transfected cell line

On the day before transfection, the density of CHO-K1 cells was adjusted to $0.5 \times 10^6$ cells/mL. On the day of transfection, high concentration endotoxin-free plasmids that had undergone linear treatment were prepared, the cell density and viability of CHO-K1 cells were measured, ensuring that the cell viability was greater than 97%. After the CHO-K1 cells were washed twice with CD CHO medium, an electroporation transfection reaction system was prepared: 700 μL of cell suspension and 40 μg of plasmid were mixed well and transferred into a 4 mm of electrode cup. The electrode cup was placed into an electroporator and the shock parameters were set to 300 V, 1000 μF. After one electric shock, the shocked cell suspension was transfer into preheated fresh CD CHO medium, and incubated at 37 °C for 20 minutes. The incubated cell suspension was inoculated evenly into a 96-well plate. 24 hours after the transfection, the cells were pressurized, added with CD CHO medium containing methionine sulfoximine (MSX) with the final concentration of 25-50 μM, and statically cultured under 5% $CO_2$, at 37 °C.

### Example 2: Screening of Monoclonal Cell Lines with High Expression

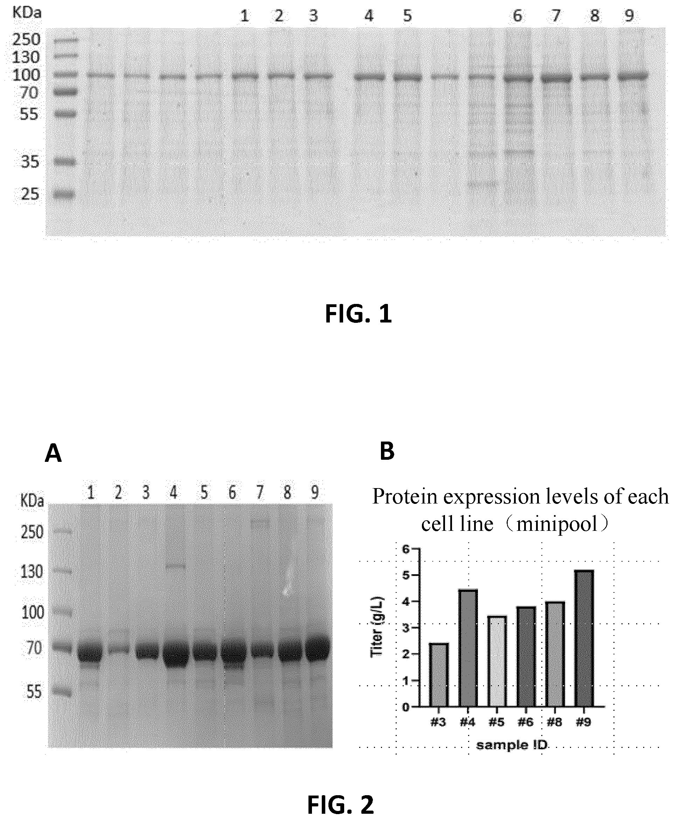

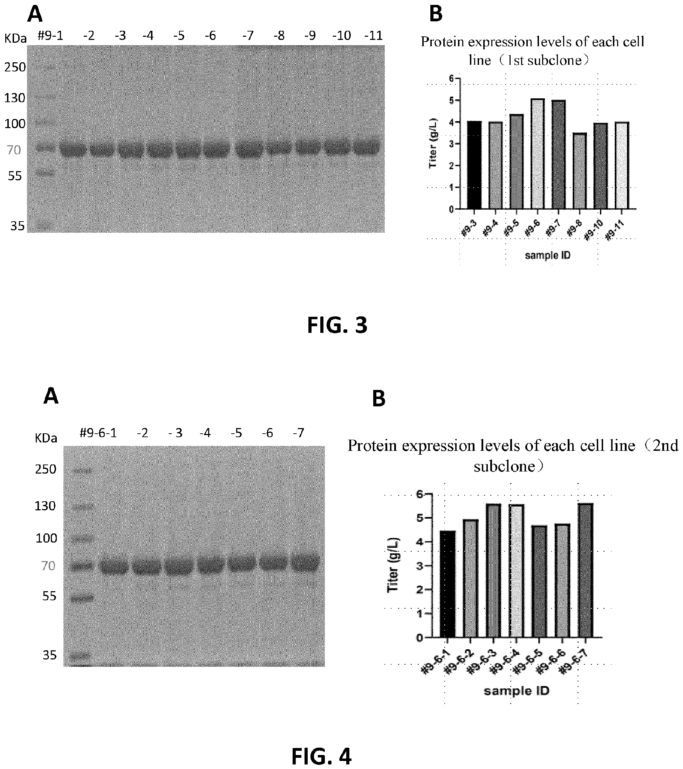

After growing to an appropriate size in the 96-well plate, the monoclonal cells were selected and transferred to anew 96-well plate. The selected monoclonal cells were incubated in 5% $CO_2$ at 37 °C for static culture. After the cells in the well grew to confluency, the supernatant was taken from the well plate for reducing electrophoresis to detect the expression of the fusion proteins. Nine clones with the highest expression levels were selected (see FIG. 1) and gradually expanded to shake flask culture. Nine clones were subjected to fed-batch culture in 25 mL shake flasks. The culture supernatant was harvested and identified by non-reducing electrophoresis (see FIG. 2A) and calculated for the protein expression level of each cell line (see FIG. 2B). The cell line # 9 with the highest expression level was selected. Cell line # 9 was screened for the monoclonal cell lines by using limited dilution approach. The cells were inoculated in 96-well plates with 0.3 cells/well to obtain 11 cell lines having high expression. These cell lines were then subjected to fed-batch culture in 25 mL shake flasks. The supernatants were identified by non-reducing electrophoresis (see FIG. 3A) and calculated for the protein expression level of each cell line (see FIG. 3B). The cell line #9-6 with the highest expression level was selected. The cell line #9-6 were further screened for monoclonal cell lines by using the limited dilution approach, resulting in 7 cell lines with high expression level. The obtained cell lines were subject to fed-batch culture in 25 mL shake flasks. The supernatants were identified by non-reducing electrophoresis (see FIG. 4A) and calculated for the protein expression level of each cell line (see FIG. 4B). The cell line #9-6-7 with high stability and expression level was selected as cell line IL-2-HSA/CHOK1 with stable and high expression of the protein.

### Example 3: Fed-Batch Culture of the Stable Cell Line in 125 mL Shake Flask

On the day (recorded as D0) of starting the cultur for expression, IL-2-HSA/CHOK1 cells in 25 mL of basal medium containing 25-50 μM MSX were inoculated into a 125 mL of shake flask at a density of $0.3 \times 10^6$ cells/mL, and cultured with 5% $CO_2$ at 37 °C on a shaker at 135 rpm. On D4 of inoculation, the samples were taken and counted, and the culture temperature was decreased to 33 °C when the cell density reached to $10 \times 10^6$ cells/mL. On D5, fed-batch culture was carried out and the glucose concentration was controlled to 3 ~ 4 g/L. On D13 of culture, the culture was terminated, the cell culture supernatant was harvested. Then, the expression level of the fusion protein was measured to be 4.36 mg/mL.

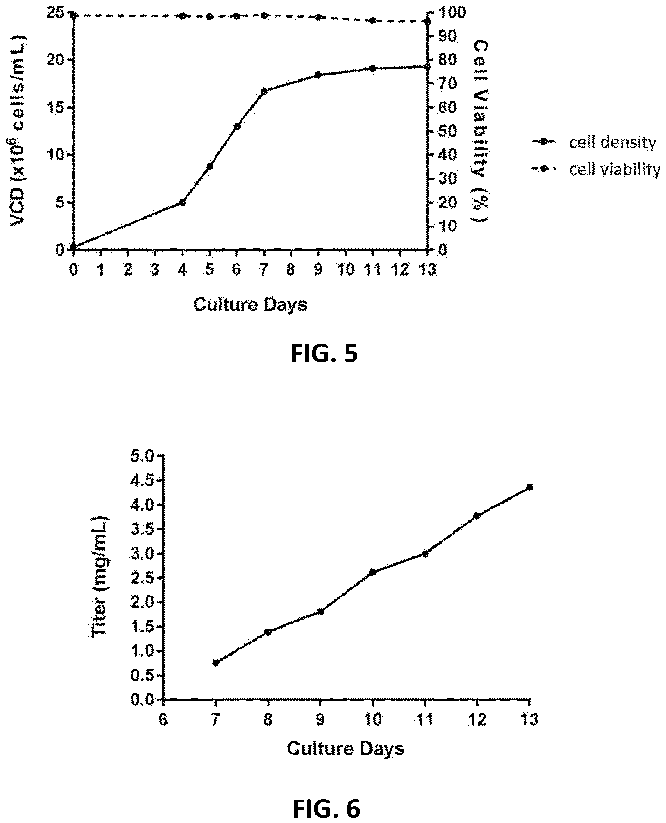

A culture kinetics curve of IL-2-HSA/CHOK1 cells is shown in FIG. 5. It can be seen from FIG. 5 that in the early stage of culture, the cells were in a logarithmic growth stage, with a rapid increase in density; in the later stage of culture, the cells entered a protein production stage, and the cell density tended to be stable. The maximum live cell density reached to $19.3 \times 10^6$ cells/mL.

A kinetics curve of the expression level of IL-2-HSA/CHOK1 cell supernatant is shown in FIG. 6. It can be seen from FIG. 6 that from D7 to D13, with the increase of culture days, the protein expression level of the cells showed a gradually increasing trend. The expression level on D13 was 4.36 mg/mL.

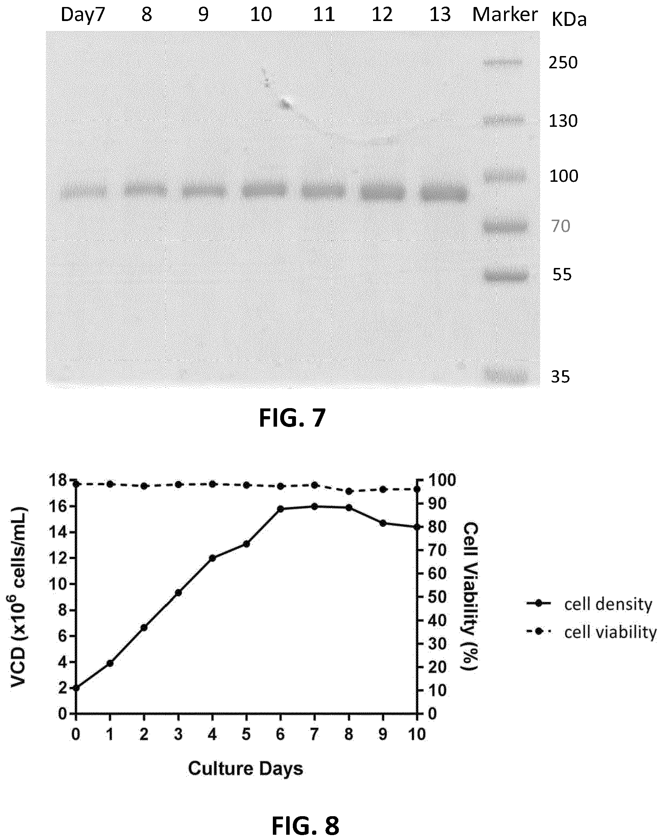

A non-reducing electrophoresis analysis of the protein expression of IL-2-HSA/CHOK1 cell supernatant is shown in FIG. 7. It can be seen from FIG. 7 that from D7 to D13, with the increase of culture days, the protein expression level of the cells showed a gradually increasing trend. The target protein had a single band without impurities.

**Example 4: Fed-Batch Culture of the Stable Cell Line in 1 L of Shake Flask**

IL-2-HSA/CHOK1 cells were inoculated into a 50 mL of shaking flask at a density of $2 \times 10^6$ cells/mL, and cultured with 5% $CO_2$ at 37 °C on a shaker at 135 rpm. The samples were taken and counted daily to observe cell status, and the basal medium containing 25~50 $\mu$M MSX was supplemented. The cell density was daily adjusted to $2 \times 10^6$ cells/mL until the volume of the cell culture solution reached to 300 mL. Then, stop supplement the basal medium and continue the culture, at which point it was recorded as D0. The samples were taken and counted daily and 1 mL of culture supernatant was retained. The culture temperature was decreased to 33 °C when the cell density reached to $6 \times 10^6 \sim 7 \times 10^6$ cells/mL. On D2, the fed-batch culture was started and the glucose concentration was controlled to 3 g/L. On D10 of culture, the culture was terminated, the cell culture supernatants were harvested, and the protein expression levels of the supernatants were measured from D0 to D10. The expression level of fusion protein in the supernatant harvested on D10 was measured to be 3.12 mg/mL.

A kinetics curve of IL-2-HSA/CHOK1 cell culture is shown in FIG. 8. It can be seen from FIG. 8 that in the early stage of culture, the cells were in a logarithmic growth stage, with a rapid increase in density; in the later stage of culture, the cells entered a protein production stage, and the cell density tended to be stable. The maximum live cell density reached to $16 \times 10^6$ cells/mL.

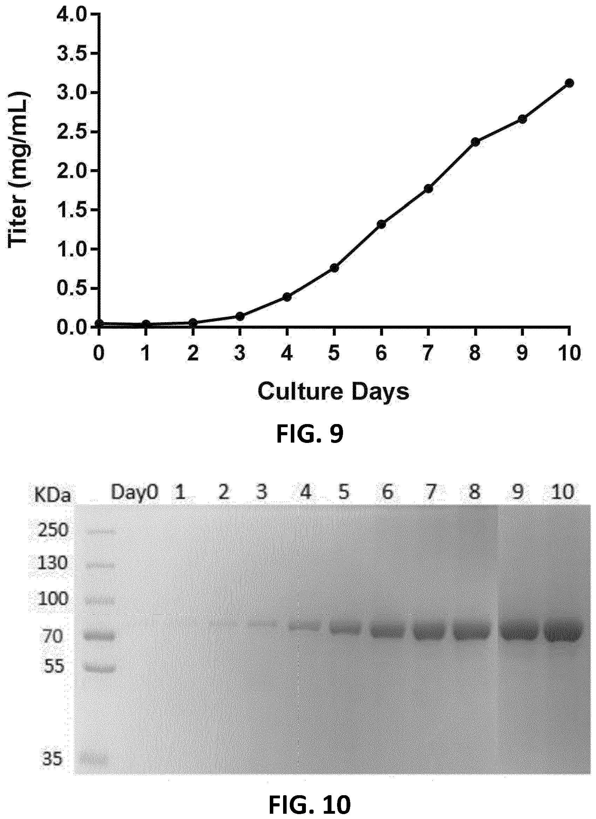

A kinetics curve of the expression level of IL-2-HSA/CHOK1 cell supernatant is shown in FIG. 9. It can be seen from FIG. 9 that with the increase of culture days, the protein expression level of the cell showed a gradually increasing trend.

A non-reducing electrophoresis analysis of the protein expression of IL-2-HSA/CHOK1 cell supernatant is shown in FIG. 10. It can be seen from FIG. 10 that with the increase of culture days, the protein expression level of the cells showed a gradually increasing trend. The target protein had a single band and no impurities.

Compared with the fed-batch culture in shake flasks in the prior art, the cell density and cell survival rate of the present disclosure are very high, as shown in FIG. 8. The cell density reaches to $14 \times 10^6 \sim 16 \times 10^6$ cells/mL on D6 to D10. The protein expression level of the cells of the present disclosure is also very high, as shown in FIG. 9. The expression level on D10 is 3.12 mg/mL. In the present disclosure, even at the cell density of $14 \times 10^6 \sim 16 \times 10^6$ cells/mL, the expression level is also high, indicating that the cells remain high activity at such a cell density.

**Example 5: Assay of Effect of IL-2-HAS on NK-92 Cell Proliferation**

In this example, NK-92 cells (ATCC® CRL-2407 ™, an IL-2 dependent NK cell line derived from the peripheral blood monocytes of a 50 year-old male patient with malignant non-Hodgkin's lymphoma) were utilized to evaluate the biological activity of IL-2-HSA.

(1) Cryopreserved NK-92 cells were taken out from a liquid nitrogen tank, thawed and cultured until reaching the logarithmic growth phase.

(2) Sufficient cells were collected by centrifugation, resuspended in the complete culture medium without IL-2, and cultured under starvation for 24 hours.

(3) The starved NK-92 cells were centrifuged, resuspended in the complete culture medium without IL-2, and counted. The cell density was adjusted to $5 \times 10^5$ cells/mL, and the cells were added into the 96-well plate at a volume of 90 $\mu$L per well.

(4) Preparation of sample solution: IL-2-HSA and rhIL-2 (R&D, Cat. No. 202-IL) samples were pre-diluted to 50.67 nM with the medium, and diluted to 9 concentrations at a 4-fold gradient. The diluted samples were added to corresponding wells of the 96-well plate at 10 $\mu$L/well, with three replicates for each concentration. For the negative control group, the medium was added at 10 μL/well correspondingly and mixed well.

(5) After culturing under 5% $CO_2$ at 37 °C for 72 hours, the melted and mixed MTS detection reagent was added to the above 96-well plate at 20 μL/well, shook well in an oscillator, and then incubated in a cell culture incubator at 37 °C, 5% $CO_2$ for additional 1 to 4 hours.

(6) After incubation, shook and mixed well. The absorbance was measured at a wavelength of 490 nm using a microplate reader.

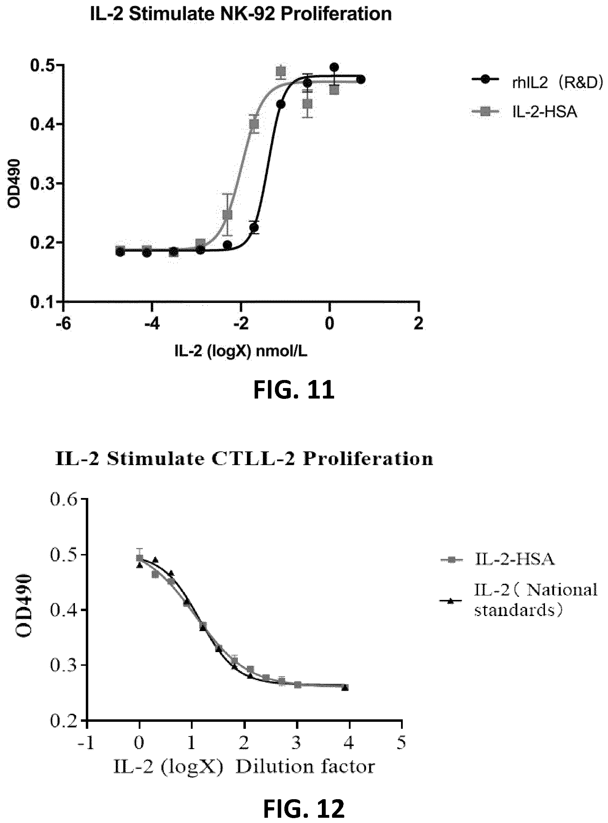

(7) The data was analyzed using GraphPad Prism 8 software, with the logarithm of drug concentration X as the horizontal coordinate and $OD_{490}$ as the vertical coordinate. The drug action curve was fitted with four parameters. The $EC_{50}$ values obtained are shown in Table 1, and the proliferation curve is shown in FIG. 11.

Table 1

|  | IL-2 (R&D) | IL-2-HSA |
|---|---|---|
| $EC_{50}$ (nM) | 0.0420 | 0.01047 |

It can be seen from FIG. 11 that IL-2-HSA induced NK-92 cell growth in a dose-dependent manner. Under the experimental condition, the activity of IL-2-HSA in stimulating NK-92 proliferation was higher than that of equimolar rhIL-2 (about 4 times).

**Example 6: Assay of Effect of IL-2-HAS on CTLL-2 Cell Proliferation**

In this example, CTLL-2 cells (ATCC® TIB™, a mouse cytotoxic T lymphocyte cell line, IL-2 dependent) were utilized to evaluate the biological activity of IL-2-HSA.

Referring to the human interleukin-2 biological activity assay (CTLL-2 cell/MTT colorimetric assay) in the Chinese Pharmacopoeia, CTLL-2 cells were inoculated into 96-well plate at a density of 30000 cells/well, and added with a series of gradient diluted national standards and IL-2-HSA, and cultured in 5% $CO_2$ at 37 °C for 18 to 24 hours. Then, MTS reagent was added and incubated for 1 to 4 hours. After shaking and mixing well, the absorbance was measured at a wavelength of 490 nm using a microplate reader. The data was analyzed using GraphPad Prism 8 software, with the logarithm of dilution X as the horizontal coordinate and $OD_{490}$ as the vertical coordinate. The drug action curve was fitted with four parameters. The $EC_{50}$ values obtained are shown in Table 2, and the proliferation curve is shown in FIG. 12.

The biological activity of IL-2-HSA is calculated using the following formula:

$$\text{biological activity of test sample IU/mL}$$
$$= \text{biological activity of standard IU/mL} \times \frac{\text{pre} - \text{dilution ratio of test sample}}{\text{pre} - \text{dilution ratio of standard}}$$
$$\times \frac{\text{dilution ratio of test sample equivalent to half effective amount of standard}}{\text{dilution ratio of semi} - \text{effective amount of standard}}$$

After calculation, the specific activity of IL-2-HSA was $8.38 \times 10^6$ IU/mg, comparable to the specific activity (not less than $1 \times 10^7$ IU/mg) of IL-2 as specified in the pharmacopoeia. However, the difference between the molecular weights of IL-2-HAS and IL-2 was about 5 times. Thus, the specific activity of IL-2-HSA was higher.

Table 2

|  | IL-2 (National standard) | IL-2-HSA |
|---|---|---|
| $EC_{50}$(dilution ratio) | 13.90 | 11.68 |

**Example 7: Preventive and therapeutic effects of the IL-2-HSA fusion protein on ulcerative colitis mouse model induced by DSS**

**1. Animal selection and grouping, model establishment, and dosing regimen:**

108 male C57BL / 6 wild-type mice (7-8 weeks old) were randomly divided into 7 groups according to body weight: G1 was healthy control group, G2 was model control group, G3 was commercially available short-effect IL-2 control group ([125]Ala IL-2, $3 \times 10^4$ICT / mouse / d, s.c.), G4 were HSA control group (0.3 mg / kg / 4d, s.c.), G5 was IL-2-HSA fusion protein, low-dose group ($1 \times 10^4$IU /mouse/ 4d, s.c.), G6 was IL-2-HSA fusion protein, mid-dose group (3×10⁴IU /mouse/ 4d, s.c.), and G7 was the IL-2-HSA fusion protein, high-dose group (1×10⁵IU/mouse/4d, s.c.). There were 12 healthy mice in the control group and 16 mice in each of the other groups.

The modelled animals in each group started drinking water supplemented with dextran sodium sulfate (DSS) of 2.5% (wt / vol) on Day 2 ($D_2$), for 6 days (days 2-7 defined as DSS period), to induce colitis model. The animals started drinking water without DSS from $D_8$. The animals were s.c. administrated with corresponding drugs or sterile water before or after the model establishment. Either IL-2-HSA fusion protein or HSA was administrated every 4 days, respectively, at $D_0$, $D_4$, $D_8$, 3 times in total; [125]Ala IL-2 was administrated daily from $D_0$ to $D_8$, 9 times in total.

## 2. Testing index

### 2.1 General clinical observation

During the trial, the surviving animals were observed clinically twice a day, including, but not limited to, physical signs, mental state, behavioral activities, food- and water- intake, faecal traits, weight changes (the focus of the observation) or other abnormal conditions.

### 2.2 Weight

The animals in all of the above groups were weighed: weigh before grouping; weigh daily during the trial; weigh before euthanasia; and weigh the animals that died unexpectedly.

### 2.3 Faecal traits and hematochezia degree

From the date of modeling ($D_2$), the faecal traits and the hematochezia degree of the surviving animals in all of the above groups were scored once a day. The rules were as follows. Faecal traits: 0= normal; 1= faeces-forming slightly soft; 2= faeces-forming very soft; 3= diarrhea; 4= dysenteric diarrhea. Hematochezia degree: 0= faeces occult blood, negative; 1= faeces occult blood, positive; 2= visible blood in faeces; 3= visible rectal bleeding.

### 2.4 Determination of colon length and weight

Half of the animals in each group at the end of the DSS period ($D_8$) and the remaining animals in each group at the end of the trial, were euthanized, to remove the colon. The colon length was measured, and the colon was weighed.

### 2.5 Spleen weight determination and lymphocyte subpopulation analysis

Half of the animals in each group at the end of the DSS period ($D_8$) and the remaining animals in each group at the end of the trial, were euthanized, to aseptically remove spleen. The spleen was weighed, and single-cell suspension of the spleen was prepared. Then, the proportions of CD8$^+$ T cells, CD4$^+$ T cells and T$_{reg}$ cells (CD4$^+$CD25$^+$Foxp3$^+$) were detected by flow cytometry.

## 3. Statistical analysis

The data were described using the mean and standard deviation (Mean $\pm$ SD), plotted with GraphPad Prism 8 software and statistical analyzed with SPSS 25. The process included: perform the homogeneity of variance test on the data; perform one-way analysis of variance (One-way ANOVA on the data with homogeneity of variance ($P> 0.05$); perform LSD multiple comparison analysis on the data having testing difference ($P\leq0.05$); perform Kruskal-Wallis non-parametric test on the data without homogeneity of variance ($P\leq0.05$); perform Mann-Whitney comparison analysis on the data having testing difference ($P\leq0.05$). $P\leq0.05$ was considered as having significant difference. The differences of the testing indexes between the healthy control group and the model control group, as well as between the model control group and each treatment group were mainly investigated.

## 4. Test results

### 4.1 Animal Survival

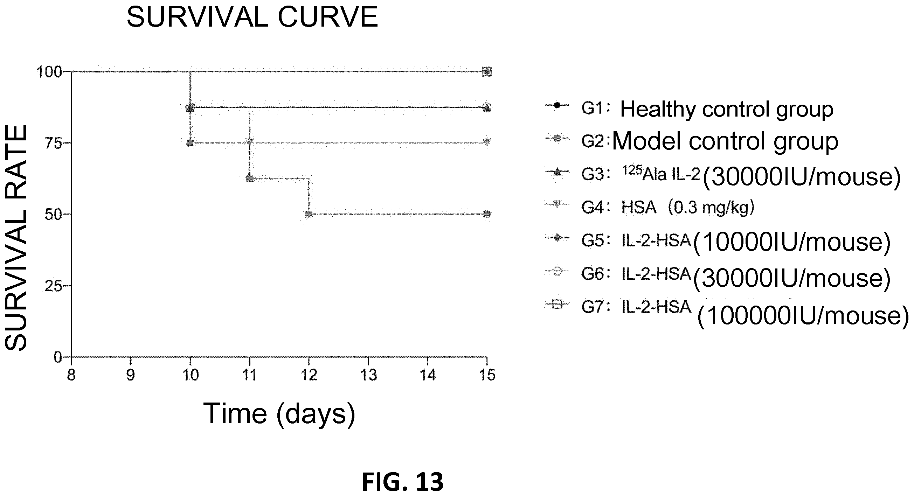

A total of 8 animals died during the recovery period ($D_8$-$D_{15}$) in the trial, which included: 4 in model control group, 1 in [125]Ala IL-2 group, 2 in HSA control, and 1 in the mid-dose group of IL-2-HSA fusion protein. At the end of the trial, the death rates of the healthy control group (G1), model control group (G2), commercially available short-acting IL-2 control group (G3), HSA group (G4), IL-2-HSA fusion protein, low-dose group (G5), IL-2-HSA-HSA fusion protein mid-dose group (G6) and IL-2-HSA fusion protein high-dose group (G7) were 0%, 50%, 12.5%, 25%, 0%, 12.5% and 0%, respectively. FIG. 13 shows the effect of the IL-2-HAS fusion protein on the survival rate of C57BL/6 mice of colonitis models induced by DSS in the recovery phase ($D_8$-$D_{15}$). As shown in FIG. 13, the death rates of the mice of colonitis models induced by DSS can be decreased by prophylactic administration of the IL-2-HAS fusion protein at $1\times10^4$IU / mouse to $1\times10^5$IU /mouse every 4 days (i.e. G5-G7: IL-2-HSA fusion protein, at low-, mid- and high-dose).

4.2 Weight

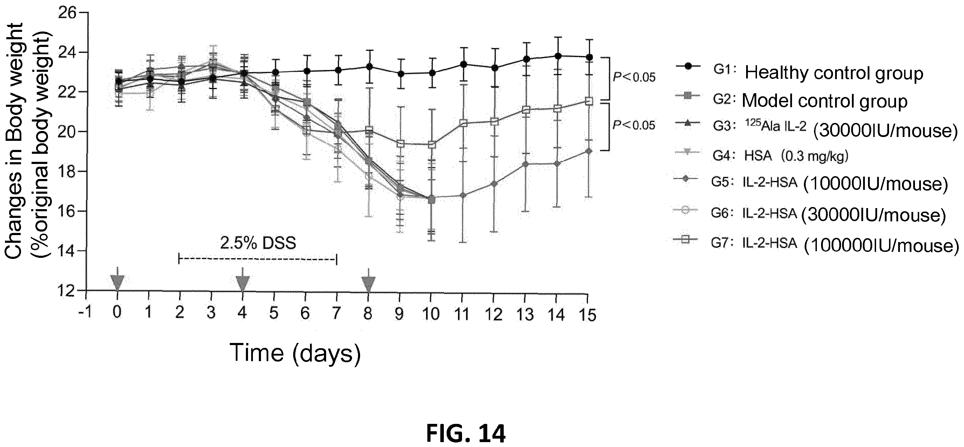

The changes of the body weight of the mice in the seven groups are shown in FIG. 14. FIG. 14 shows the effect of the IL-2-HAS fusion protein on the body weight of C57BL/6 mice of colonitis models induced by DSS. It can be seen from FIG. 14 that the body weight of the animals from healthy control (G1) fluctuantly increased during the trial. The body weight of the animals from each group (G2-G7) significantly decreased from Day 3 after drinking DSS water ($D_4$), and the animals began to die at $D_{10}$. Later, the body weight of the surviving animals rose slowly. Among them, the weight loss of the mice from the IL-2-HSA fusion protein in the high-dose group (G7) was significantly reduced, lower than that of the model control group (G2). Two-way ANOVA was performed with SPSS statistical software. The results show that the combination of treatment and time can produce significance (P <0.05).
According to FIG. 14, the IL-2-HSA fusion protein can dose-dependently improved the weight loss in mice with DSS-induced ulcerative colitis. The IL-2-HSA fusion protein can significantly inhibit the weight loss in mice with DSS-induced colitis at $1\times10^5$IU /mouse (G7, IL-2-HSA fusion protein, high-dose group).

4.3 Faecal traits and hematochezia degree score

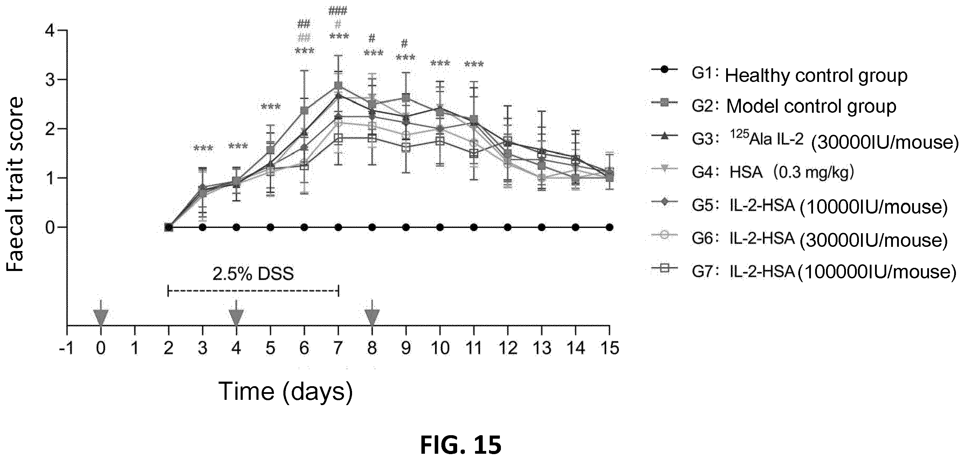

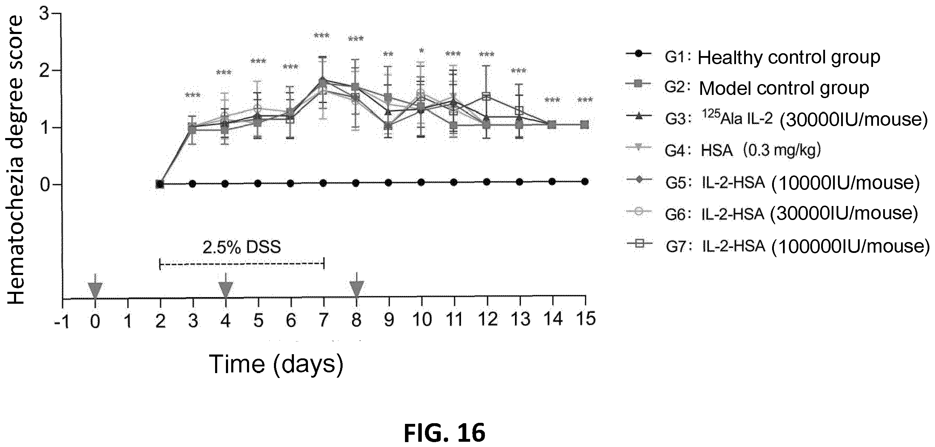

The statistical results of the scores of the faecal traits and of hematochezia degree of the mice from the seven groups are shown in FIGs. 15 and 16. FIG. 15 shows the effect of the IL-2-HAS fusion protein on the faecal traits of C57BL/6 mice of colonitis models induced by DSS. FIG. 16 shows the effect of the IL-2-HAS fusion protein on the hematochezia degree of C57BL/6 mice of colonitis models induced by DSS. It can be seen from FIGs. 15 and 16 that, compared with healthy control (G1), the animals from model control groups (G2-G7) started showing colitis-related clinical symptoms such as loose faeces and hematochezia, from Day 2 after DSS drinking ($D_3$), with increasing clinical scores, and gradually recovering after stopping DSS drinking. The animals from IL-2-HSA fusion protein low-, mid- and high-dose groups (G5-G7) started showing colitis-related clinical symptoms such as weight loss, loose faeces and hematochezia appeared, from Day 2 after DSS drinking ($D_3$), but significantly less than those in the model control group (G2). It showed a certain dose-effect relationship. Especially, the faecal trait scores were significantly reduced for the IL-2-HSA fusion protein mid-dose group (G6) at $D_6$-$D_7$, and the IL-2-HSA fusion protein high-dose group (G7) at $D_6$-$D_9$ (P <0.05).
According to FIGs. 15 and 16, it shows that the IL-2-HSA fusion protein can dose-dependently improve diarrhea in mice with DSS-induced ulcerative colitis. In particular, the IL-2-HSA fusion protein can significantly improve the clinical symptoms of the mice with DSS-induced colitis at $3\times10^4$ICT/mouse (G6) and $1\times10^5$ IU /mouse (G7).

4.4 Length and weight of the colons

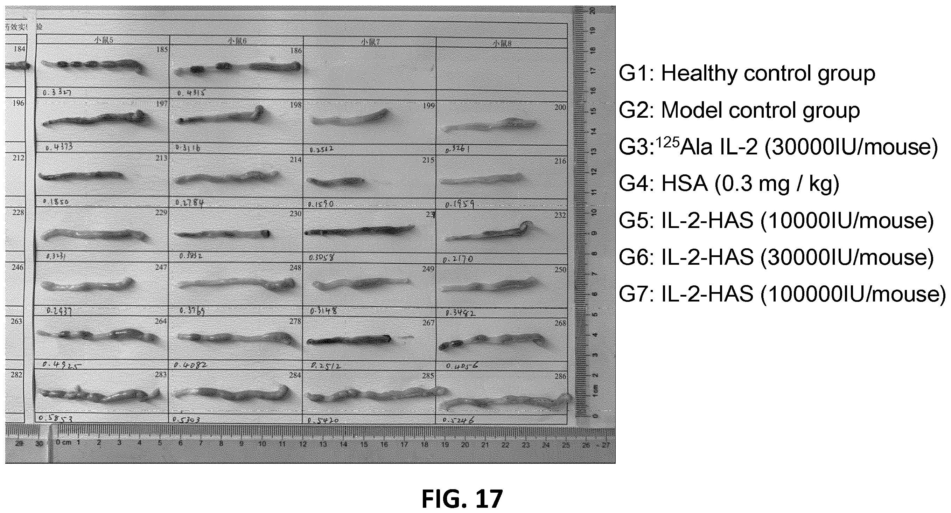

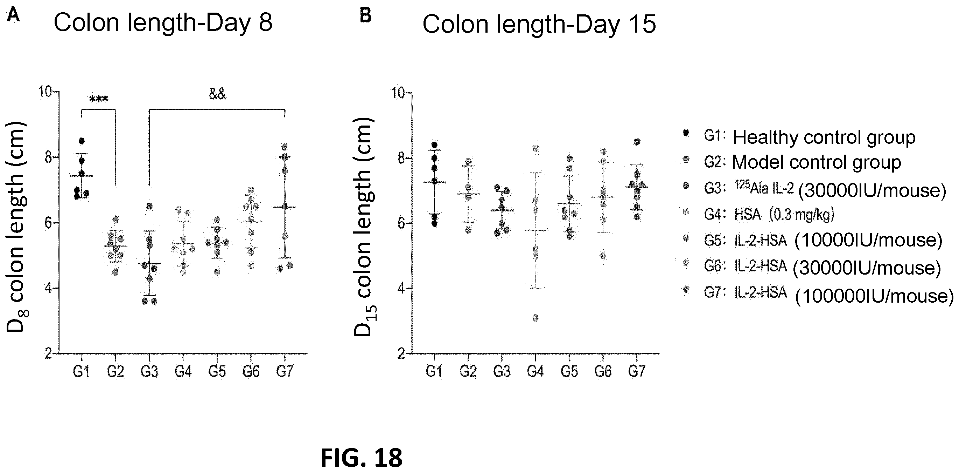

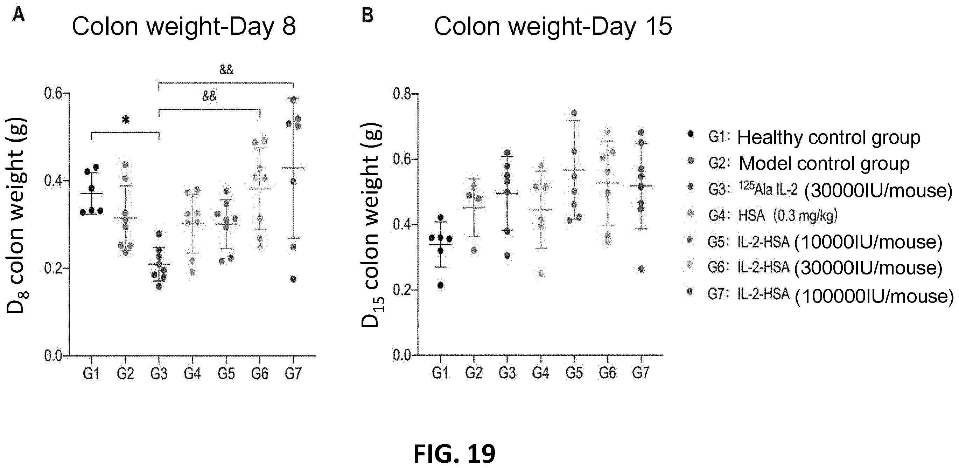

In this trial, half of the animals from each group at the end of DSS ($D_8$) and the remaining animals from each group at the end of the recovery period (D15) were evaluated for their colon tissues.
The colon samples of the animals from 7 groups were shown in FIG. 17. The colon length was shown in FIG. 18, and the colon weight was shown in FIG. 17. FIG 17 shows the photographs of the colon samples from C57BL/6 mice euthanized on D8. FIG. 18 shows the effect of the IL-2-HAS fusion protein on the colon length of C57BL/6 mice of colonitis models induced by DSS. FIG. 19 shows the effect of the IL-2-HAS fusion protein on the colon weight of C57BL/6 mice of colonitis models induced by DSS. It can be seen from FIGs. 18 and 19 that the colon length was $7.43\pm0.67$, $5.29\pm0.48$, $4.76\pm0.99$, $5.36\pm0.68$, $5.39\pm0.47$, $6.04\pm0.91$, $6.47\pm1.55$ cm, respectively, for the animals from the healthy control group (G1), model control group (G2), commercially available short-acting IL-2 control group (G3), HSA group (G4), IL-2-HSA fusion protein low-dose group (G5), IL-2-HSA fusion protein mid-dose group (G6), IL-2-HSA fusion protein high-dose group (G7) on $D_8$. The colon weight (including contents) was $0.37\pm0.05$, $0.31\pm0.07$, $0.21\pm0.04$, $0.30\pm0.07$, $0.30\pm0.06$, $0.38\pm0.09$, $0.43\pm0.16$ g, respectively. Compared with the healthy control group (G1), the colon length of model control group (G2) was significantly shortened, having statistically significant difference (P < 0.05). Compared with model control group (G2), the colonic length of IL-2-HSA fusion protein in low-, mid- and high-dose groups (G5-G7) increased in a dose-dependent manner, but having no statistically significant difference. Compared with [125]Ala IL-2 group (G3), the colon weight of IL-2-HSA-HSA fusion protein mid-dose group (G6) was significantly increased (P <0.01), and both of colon length and colon weight of the IL-2-HSA fusion protein high-dose group (G7) were significantly increased (P <0.01). The above results indicate that the IL-2-HSA fusion protein had more significant therapeutic effect than the short-acting IL-2 at the same dose ($3\times10^4$IU /mouse), having statistically significant difference. Thus, the IL-2-HSA fusion protein of the present invention not only extends the administration interval, but also has better efficacy, with respect to the existing [125]Ala IL-2.

According to FIGs. 18 and 19, the IL-2-HSA fusion protein can dose-dependently improve colon shortening of the mice with DSS-induced ulcerative colitis. Further, the IL-2-HSA fusion protein can prevent colon shortening of the mice with DSS-induced colitis at $3\times10^4$IU /mouse and $1\times10^5$ IU / mouse (G6, G7).

4.5 Spleen weight and organ coefficient

In this trial, half of the animals from each group at the end of DSS ($D_8$) and the remaining animals from each group at the end of the recovery period ($D_{15}$) were evaluated for their spleen weight.

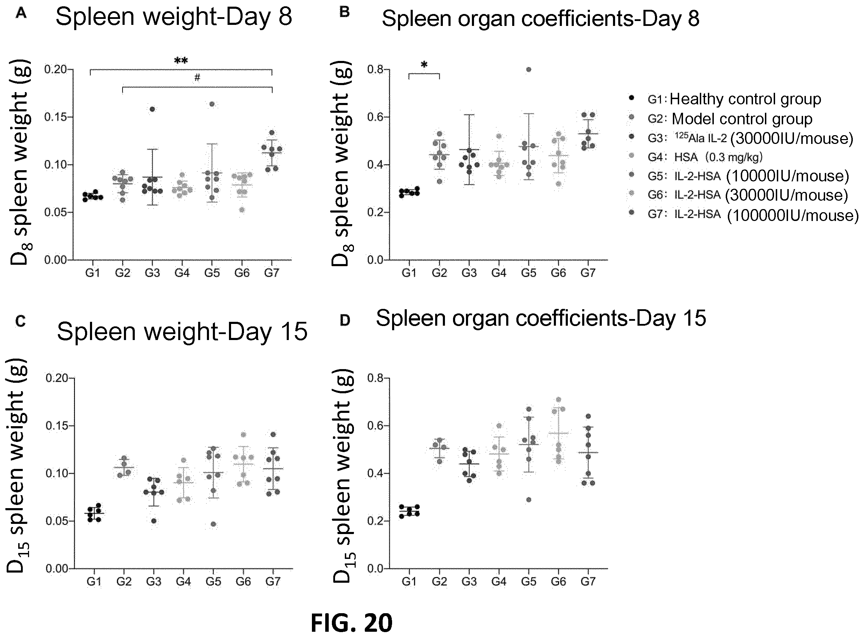

The spleen weight and spleen organ coefficients of 7 groups were shown in FIG. 20. FIG. 20 shows the effect of the IL-2-HAS fusion protein on the spleen weight and organ coefficients of C57BL/6 mice of colonitis models induced by DSS. As can be seen from FIG. 20, the spleen weight on $D_8$ of the mice from each group was similar to the model control group, except that the IL-2-HSA fusion protein high-dose group increased significantly as compared with the model control group (0.11 ± 0.01 g Vs 0.08 ± 0.01 g, P <0.05). The spleen organ coefficient on $D_8$ of the mice from model control group (G2) increased significantly as compared with healthy control (G1) (0.44 ± 0.06% Vs 0.28 ± 0.01%, P <0.05), with no significant difference between the other groups and the model control group.

4.6 Analysis of the spleen lymphocyte subpopulations

In this trial, half of the animals from each group at the end of DSS ($D_8$) and the remaining animals from each group at the end of the recovery period ($D_{15}$) were analyzed for the spleen lymphocyte subpopulations.

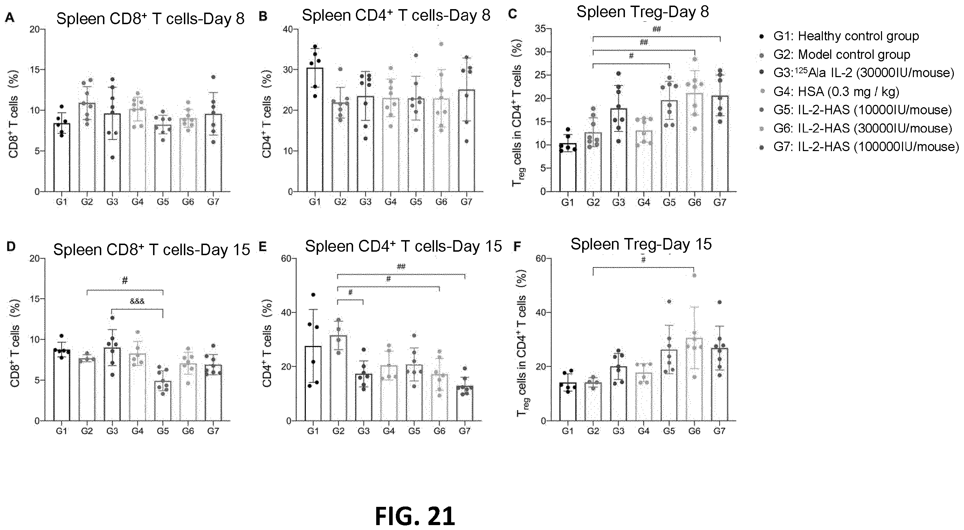

The spleen CD8$^+$ T cells, CD4$^+$ T cells and the proportions of $T_{reg}$ cells (CD4$^+$CD25$^+$Foxp3$^+$) in CD4$^+$T cells were shown in FIG. 21. FIG. 21 shows the effect of the IL-2-HAS fusion protein on the spleen lymphocyte subpopulation of C57BL/6 mice of colonitis models induced by DSS. It can obtain the followings from FIG. 21.

trial $D_8$, as compared with the model control group (G2), the proportions of spleen CD8$^+$ and CD4$^+$ T cells of the animals from the IL-2-HSA fusion protein low-, mid- and high-dose group (G5-G7) were not significantly altered; CD4$^+$CD25$^+$Foxp3$^+$(classics $T_{reg}$) subpopulations in CD4$^+$ T cells were significantly induced (P <0.05); the proportion of $T_{reg}$ subpopulation of the animals from $^{125}$Ala IL-2 group also increased to some extent.

trial $D_{15}$, as compared with the model control group, the proportions of spleen CD8$^+$ and CD4$^+$ T cells of the animals from the IL-2-HSA fusion protein low-, mid- and high-dose group decreased, but the proportion of $T_{reg}$ subpopulation in CD4$^+$T cells remained at a high level.

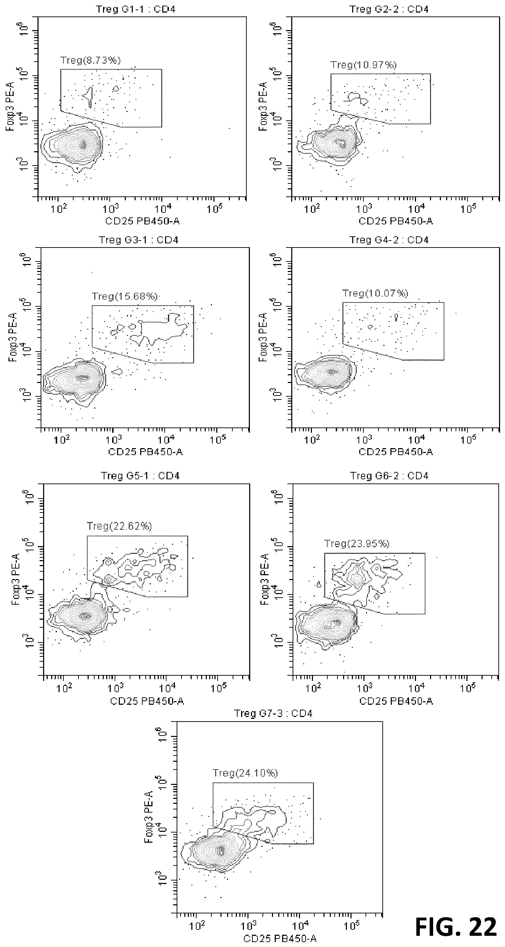

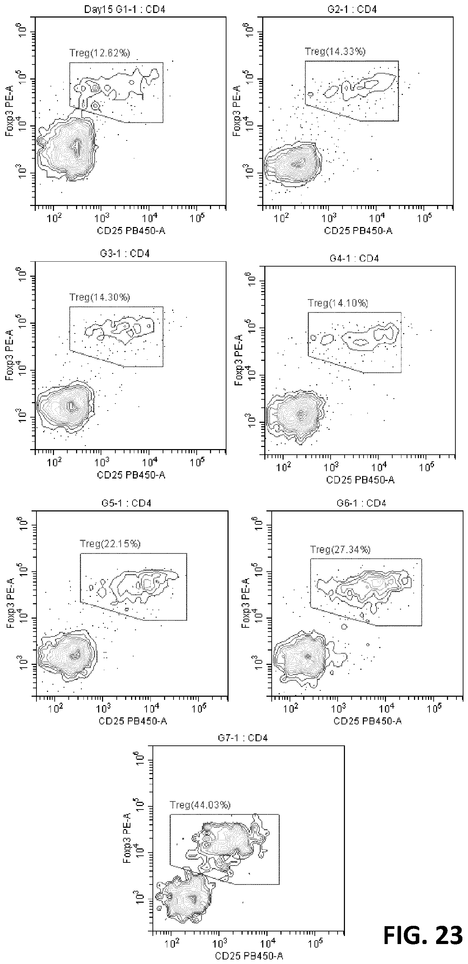

Representative results of $T_{reg}$ cell on $D_8$ and $D_{15}$ from 7 groups are shown in FIGs. 22 and 23. FIG. 22 shows the representative flow cytometry results ($D_8$) of the spleen $T_{reg}$ (CD4$^+$CD25$^+$Foxp3$^+$) cells from the mice of colonitis models. FIG. 23 shows the representative flow cytometry results ($D_{15}$) of the spleen $T_{reg}$ (CD4$^+$CD25$^+$Foxp3$^+$) cells from the mice of colonitis models.

According to FIGs. 22 and 23, the IL-2-HSA fusion protein can permanently increase peripheral $T_{reg}$ cell levels in mice and contribute to the reduction of CD8$^+$T cytotoxic effect at $1\times10^4$IU / mouse to $1\times10^4$ IU / mouse.

**Example 8: Affinity analysis of the IL-2-HSA fusion protein with IL-2Rs of different species**

The interaction between the IL-2-HSA fusion protein and IL-2 receptors (IL-2Rs) of different species (human, dog, rat, mouse) were analyzed by Bio-Layer Interferometry (BLI). IL-2Rs of different species were captured through ProA or hFc probes. The IL-2-HSA fusion protein was diluted to different concentration gradients. Then, the association and dissociation of the IL-2-HSA fusion protein with the IL-2Rs of different species attached to ProA or hFc probes were measured, to determine the affinity of the IL-2-HSA fusion protein with the IL-2Rs of different species. The specific processes are described as follows:

(1) Human IL-2R (hIL2R, BIOSYSTEMS, ILGH5257) and mouse IL-2R (mIL2R, BIOSYSTEMS, ILGM5253) were diluted to 30 nM and adsorbated to ProA probe at 400 rpm/min. Rat IL-2R (rIL2R, self-made, 20220111, gene accession number: IL2Rα_RAT P26897, IL2Rβ RAT P26896, IL2RγAAH79343.1) and dog IL-2R (CaIL2R, self-made, 20220421, gene accession number: IL2Rα_O62802, IL-2RβF1PGA6, IL-2Ry_P40321) were diluted to 30 nM and adsorbated to hFc probe at 400 rpm /min.

(2) The IL-2-HSA fusion protein was diluted from 20 nM to 5 concentration gradients at a fold. A program was set so that the diluted IL-2-HSA fusion proteins was bound to the IL-2Rs attached to ProA or hFc probes, respectively, at fixed time. After the binding, the probes attached with the complex were then transferred to Q buffer without the analyte to dissociate the binding analyte.

(3) The probes were dipped into Regeneration buffer to remove residual binding analyte.

(4) The regenerated probes were placed in a 15% sucrose protective solution, and then stored at room temperature.

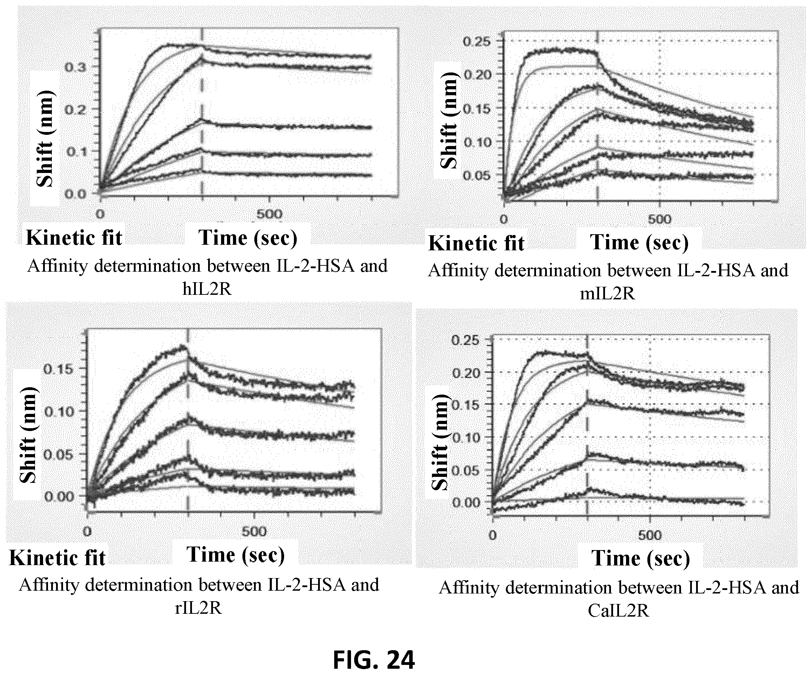

The binding abilities of the IL-2-HSA fusion protein with the IL-2Rs of different species are shown in FIG. 24 and Table 3.

Table 3

| Receptor | Sample Book | $k_{off}$(1/s) | $k_{on}$(1/Ms) | KD (M) |
|---|---|---|---|---|
| hIL2R | IL-2-HSA | 0.000169 | 1.96E+06 | 0.86E-10 |
| rIL2R | IL-2-HSA | 0.000549 | 1.77E+06 | 3.11E-10 |
| mIL2R | IL-2-HSA | 0.000887 | 1.65E+06 | 5.38E-10 |
| CaIL2R | IL-2-HSA | 0.000412 | 3.16E+06 | 1.30E-10 |

The above results indicate that: 1) the IL-2-HSA fusion protein binds to all the IL-2Rs of human, rat, mouse and dog, and has cross-species property; 2) the affinity of the IL-2-HSA fusion protein with the IL-2Rs of different species is ranked as follows: human> dog> rat> mouse.

**Example 9: Effect of the IL-2-HSA fusion protein on the *in vitro* proliferation of $T_{reg}$ cells from PBMC of healthy human**

In this example, the effect on the *in vitro* proliferation of $T_{reg}$ (CD3+CD4+CD25+CD127[low/-]) subpopulation from peripheral blood mononuclear cells (PBMCs) of healthy people of the IL-2-HSA fusion protein was compared with that of commercial rhIL-2 (R&D, Cat. No.: 202-IL) by using flow cytometry.

**1. Extraction of PBMC from human blood**

Fresh anticoagulant blood was collected from healthy people, and PBMCs were extracted with lymphocyte isolation solution (Ficoll-Paque PREMIUM, Cytiva, Cat. No.: 17-5442-02). 3-5 mL of lymphocyte isolation solution was added to a 15 mL centrifuge tube, the diluted blood sample (3 mL peripheral blood and 3 mL PBS mixed) was carefully added to the upper layer of the lymphocyte isolation solution, centrifuged at 400 g for 30-40 min. The layer of white blood cells were removed, washed twice with PBS, resuspended in RPMI-1640 medium + 10% FBS and counted.

**2. Drug treatment**

1) Cell density was adjusted to $2 \times 10^6$ cells/ mL with culture medium, inoculated in a 12-well plate at 1800 μL per well.

2) The IL-2-HSA fusion protein samples and rhIL-2 were prediluted to 10 μM with culture medium, diluted to six concentrations at a 10-fold gradient, and added to the corresponding wells of the 12-well plate at 200 μL/well. The culture medium was added to blank control wells accordingly at 200 μL/well.

3) Place in a cell culture incubator at 37°C, 5% $CO_2$, and grow continuously for 72 h.

**3. Flow-cytometric analysis**

1) Transfer the cell suspension to a new Ep tube, $2 \times 10^6$ cells/100 μL/tube.

2) Add PB450 anti-human CD3 (BD, Cat. No.: 558124,1:200), PC5.5 anti-human CD4 (BD, Cat. No.: 556924, 1:100), APC anti-human CD25 (BD, Cat. No.: 555434,1:50), PE anti-human CD127 (BD, Cat. No.: 557938, 1:100) respectively at a corresponding portion. Addtionally set negative control, monopositive control and isotype control. Incubate at 4°C for 30 min in dark place.

3) Centrifuge at 400 g for 5 min and wash twice with DPBS.

4) Resuspend with 300 μL DPBS, followed by testing on flow cytometer.

**4. Test results**

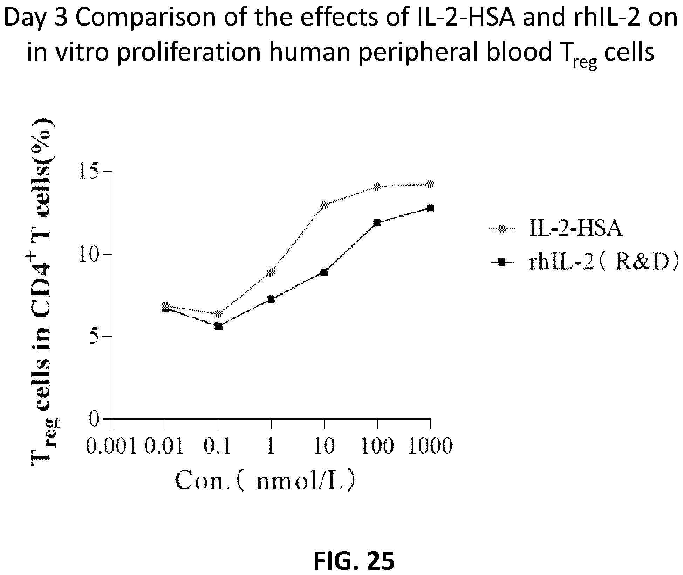

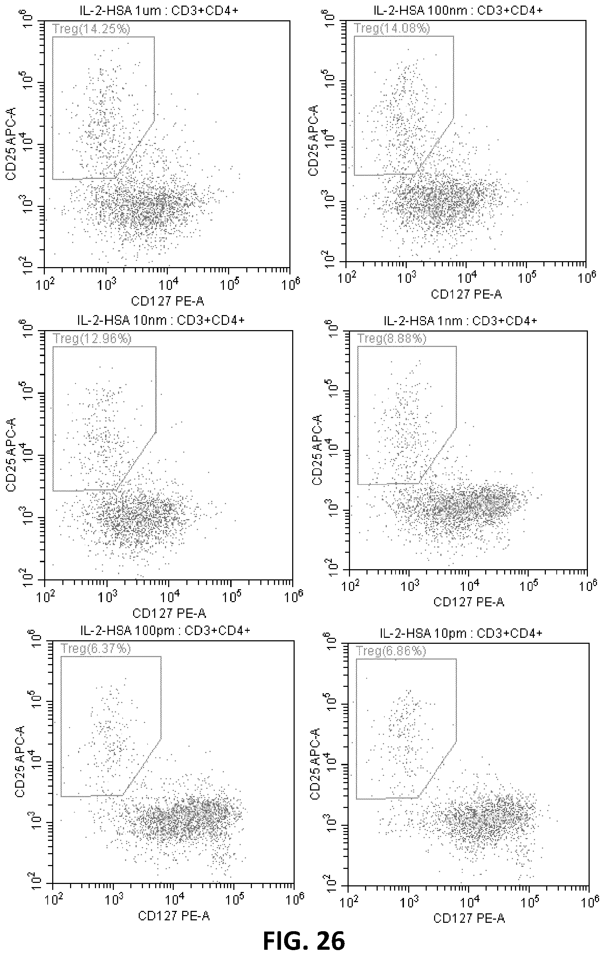

The effects of the IL-2-HSA fusion protein and rhIL-2 on the *in vitro* proliferation of $T_{reg}$ (CD3+CD4+CD25+CD1271$^{low/-}$) subpopulation in human PBMC are shown in FIGs. 25 and 26. FIG. 25 shows the comparison of the effects of the IL-2-HSA fusion protein and rhIL-2 on the proliferation of $T_{reg}$ cells in human PBMC on Day 3. FIG. 26 shows the flow-cytometric analysis results of $T_{reg}$ (CD3$^+$CD4$^+$CD25$^+$CD127$^{low/-}$) subpopulation in human PBMC.

According to the results as shown in FIG. 25, the proportion of $T_{reg}$ cells in human PBMC with increases in a dose-dependent manner after treating with the IL-2-HSA fusion protein for 72 h. In addition, the IL-2-HSA fusion protein has biological activity of promoting the proliferation of human $T_{reg}$ cell superior to rhIL-2.

**Example 10: Pharmacokinetics of the IL-2-HSA fusion protein in rat and dog with a single subcutaneous injection.**

In this example, the pharmacokinetics (PK) of the IL-2-HSA fusion protein was investigated in SD rats and Beagle dogs.

**(1) Pharmacokinetics of the IL-2-HSA fusion protein after a single subcutaneous and intravenous administration in SD rats.**

The trial included 40 SD rats, which had no administration history, and were randomly divided into 4 groups of 10 rats each, half male and half female. The animals in Groups 1-3 were administered with a single subcutaneous injection on the nape of neck. The low-, medium- and high-dosing groups were administered with $5\times10^5$ IU/kg, $1\times10^6$ IU/kg, $2\times10^6$ IU/kg, respectively. The animals in Group 4 were administered with tail intravenous injection at $1\times10^6$ IU / kg. All of the volumes of administration were 2 mL/kg. PK blood samples were collected from the test animals at the following time points:

Groups 1-3 (subcutaneous injection group) : before administration, and 1h, 2 h, 4 h, 8 h, 12 h, 24 h, 30 h, 36 h, 48 h, 72 h after administration;

Group 4 (intravenous injection group): before administration, and 2 min, 15 min, 1 h, 2 h, 4 h, 8 h, 12 h, 24 h, 48 h, 48 h, 72 h, 96 h after administration.

The concentrations of IL-2-HSA in the serum of SD rats were quantified by validated ELISA test. The pharmacokinetic parameters were calculated using noncompartmental analysis of WinNonlin software (Phoenix™, Version 8.1).

The major pharmacokinetic parameters are shown in Table 4, after the SD rats were administered with a single subcutaneous injection of different dosages ($5\times10^5$, $1\times10^6$, $2\times10^6$ IU/kg) of and a single intravenous injection of $1\times10^6$ IU/kg of the IL-2-HSA fusion protein.

**(2) Pharmacokinetics of the IL-2-HSA fusion protein after a single subcutaneous and intravenous injection in Beagle dogs.**

The trial included 24 Beagle dogs, which had no administration history, and were randomly divided into 4 groups of 6 dogs each, half male and half female. The animals in Groups 1-3 were administered with a single subcutaneous injection on the nape of neck. The low-, medium- and high-dosing group administered with $3\times10^5$ IU/kg, $6\times10^5$ IU/kg, $1.2\times10^6$ IU/kg, respectively. All of the volumes of administration were 0.6 mL/kg. The animals in Group 4 were administered with intravenous injection at $6\times10^5$ IU/kg. PK blood samples were collected from the test animals at the following time points:

Groups 1-3 (subcutaneous injection group): before administration, and 1h, 2 h, 4 h, 8 h, 12 h, 24 h, 36 h, 48 h, 60 h, 72 h, 96 h and 120 h after administration;

Group 4 (intravenous injection group): before administration, and 2 min, 15 min, 1h, 2 h, 4 h, 8 h, 12 h, 24 h, 48 h, 72 h, 96 h and 120 h after administration.

The concentrations of IL-2-HSA in the serum of Beagle dogs were quantified by validated ELISA test. The pharmacokinetic parameters were calculated using noncompartmental analysis of WinNonlin software (Phoenix™, Version 8.1).

The major pharmacokinetic parameters are shown in Table 5, after the Beagle dogs were administered with a single subcutaneous injection of different dosages ($3 \times 10^5$, $6 \times 10^5$, $1.2 \times 10^6$IU/kg) of and a single intravenous injection of $6 \times 10^5$ IU/kg of the IL-2-HSA fusion protein.

Table 4

| Species | SD rats | | | | | | | |
|---|---|---|---|---|---|---|---|---|
| Administration Route | Subcutaneous Injection | | | | | | Intravenous Injection | |
| Administration Dosage | $5 \times 10^5$IU/kg | | $1 \times 10^6$IU/kg | | $2 \times 10^6$IU/kg | | $1 \times 10^6$IU/kg | |
| Gender | female | male | female | male | female | male | female | male |
| PK parameters (mean, n =5) | | | | | | | | |
| $T_{1/2}$(h) | 3.21 | 4.21 | 3.72 | 3.78 | 4.52 | 3.82 | 5.24 | 5.40 |
| $T_{max}$(h) | 12.0 | 12.8 | 10.4 | 16.8 | 12.0 | 16.8 | - | - |
| $C_{max}$(n g/mL) | 180 | 145 | 410 | 269 | 939 | 556 | 5980 | 6362 |
| $AUC_{last}$(h*ng/mL) | 3376 | 2946 | 8593 | 5602 | 18787 | 13339 | 41544 | 47666 |
| Vd(mL/kg ) | 150 | 181 | 138 | 244 | 152 | 184 | 38.4 | 33.9 |
| CL (mL/h/kg ) | 31.9 | 30.6 | 25.4 | 42.0 | 23.6 | 32.1 | 5.12 | 4.40 |
| $MRT_{last}$(h) | 14.0 | 14.8 | 14.5 | 17.3 | 15.8 | 18.3 | 6.41 | 6.77 |

Table 5

| species | Beagle dogs | | | | | | | |
|---|---|---|---|---|---|---|---|---|
| Administration Route | Subcutaneous injection | | | | | | Intravenous Injection | |
| Administration Dosage | $3 \times 10^5$IU/kg | | $6 \times 10^5$IU/kg | | $1.2 \times 10^6$IU/kg | | $6 \times 10^5$IU/kg | |
| Gender | female | male | female | male | female | male | female | male |
| PK parameter (mean, n =3) | | | | | | | | |
| $T_{1/2}$(h) | 6.56 | 6.69 | 4.05 | 5.71 | 5.33 | 7.69 | 9.90 | 8.56 |
| $T_{max}$(h) | 10.7 | 6.67 | 8.00 | 13.3 | 13.3 | 24.0 | - | - |
| $C_{max}$(n g/mL) | 178 | 142 | 611 | 456 | 1183 | 971 | 2240 | 2073 |
| $AUC_{last}$(h*ng/mL ) | 5040 | 4334 | 18022 | 15440 | 43473 | 39360 | 33646 | 27584 |
| Vd(mL/kg ) | 120 | 157 | 43.2 | 70.8 | 44.6 | 72.5 | 54.8 | 57.8 |
| CL (mL/h/kg ) | 12.7 | 15.6 | 7.36 | 8.60 | 6.08 | 6.48 | 3.82 | 4.68 |
| $MRT_{last}$(h) | 19.6 | 21.1 | 19.5 | 21.9 | 25.1 | 26.5 | 13.2 | 12.5 |

Pharmacokinetic studies have shown that the half-life of IL-2-HSA in rats and dogs is significantly longer compared with that of natural IL-2 (several minutes in the human body). Thus, the frequency of administration and the total administration dosage can be reduced, the adverse reactions and treatment costs can be decreased, thereby improving the compliance and quality of life of patients.

The above describes the preferred embodiments of the invention in detail. However, the invention is not limited to the specific embodiments. Within the scope of the conception of the invention, various simple modifications to the technical solutions of the invention can be made, and such modifications are also within the scope of protection of the invention.

It should also be indicated that, without contradiction, the specific technical features described in the above specific embodiments can be combined in any appropriate way. To avoid unnecessary duplication, the invention does not further illustrate any possible combinations.

In addition, various embodiments of the invention may also be combined arbitrarily, provided that they do not contravene the conception of the invention. They shall also be regarded as the contents disclosed by the invention.

PCT

| Printout (The original is in electronic form) | | |
|---|---|---|
| 0-1<br>0-1-1 | Form PCT/RO/134 (SAFE) INDICATIONS RELATING TO DEPOSITED MICROORGANISM OR OTHER BIOLOGICAL MATERIAL (PCT Rule 13*bis*)<br>Software version | CEPCT<br><br>Version 10.28.47 (20220706) MT/FOP 20140331/0.20.5.21 |
| 0-2 | International application No. | PCT/CN2022/106105 |
| 0-3 | Applicant's or agent's file reference | WO11358WDb |

| 1<br><br><br>1-1<br>1-2 | The indications made below relate to the deposited microorganism or other biological material referred to in the<br>description<br>on page<br>line: | <br><br><br><br>6<br>5 |
|---|---|---|
| 1-3<br>1-3-1<br>1-3-2<br>1-3-3<br>1-3-4<br><br> | IDENTIFICATION OF DEPOSIT<br>Name of depositary institution<br><br>Address of depositary institution<br><br><br>Date of deposit<br>Accession Number | China General Microbiological Culture<br><br>Collection Center<br>Institute of Microbiology Chinese Academy of Sciences, No. 3, Courtyard NO.1, West Beichen Road, Chaoyang District, Beijing, 100101<br>May 26, 2022 (26.05.2022)<br>CGMCC 45173 |
| 1-4 | ADDITIONAL INDICATIONS | |
| 1-5 | DESIGNATED STATES FOR WHICH INDICATIONS ARE MADE | All designated states |
| 1-6 | SEPARATE FURNISHING OF INDICATIONS<br>The indications listed below will be submitted to the International Bureau later | |
| For receiving Office use only | | |
| 0-4 | This sheet was received with the international application (Yes or No) | |
| 0-4-1 | Authorized officer | |
| For International Bureau use only | | |
| 0-5 | This sheet was received by the International Bureau on | |

| Printout (The original is in electronic form) | |
| --- | --- |
| 0-5-1 | Authorized officer | |

**Claims**

1. A fusion protein of interleukin 2, comprising: a human interleukin 2 or its variant; and human serum albumin or its variant, wherein

   the human interleukin 2 or its variants includes: an amino acid sequence as shown in SEQ ID NO:1; or, an amino acid sequence having at least 90% sequence identity with the amino acid sequence as shown in SEQ ID NO:1;
   the human serum albumin or its variants includes: an amino acid sequence as shown in SEQ ID NO:2; or, an amino acid sequence having at least 90% sequence identity with the amino acid sequence shown in SEQ ID NO:2.

2. The fusion protein according to claim 1, wherein the fusion protein includes the amino acid sequence as shown in SEQ ID NO:1 and the amino acid sequence as shown in SEQ ID NO:2;

   preferably, the amino acid at position 125 of the amino acid sequence as shown in SEQ ID NO:1 is not cysteine;
   preferably, the amino acid at position 125 of the amino acid sequence as shown in SEQ ID NO:1 is replaced with serine or alanine.

3. The fusion protein according to claim 1 or 2, wherein the human interleukin 2 or its variant is connected to the human serum albumin or its variant directly or via a linking peptide.

4. The fusion protein according to claim 3, wherein the linking peptide has a general formula of $(G_nS)_m$, in which n or m is an integer selected from 1 to 10;
   preferably, the linking peptide has a general formula of $(G_nS)_m$, in which n is an integer selected from 1 to 4, and m is an integer selected from 0 to 3.

5. The fusion protein according to claim 1 or 2, wherein the fusion protein has an amino acid sequence as shown in SEQ ID NO:4.

6. An isolated nucleic acid molecule encoding the fusion protein as defined in any one of claims 1 to 5.

7. An expression system comprising a CHO cell which contains the nucleic acid molecule as defined in claim 6,

   wherein the expression system expresses the fusion protein as defined in any one of the claims 1 to 5;
   preferably, the expression system is CHO-K1 cell, which is deposited in China General Microbiological Culture Collection Center on May 26, 2022, under CGMCC No. 45173.

8. A pharmaceutical composition comprising the fusion protein as defined in any one of Claims 1 to 5, and a pharmaceutically acceptable carrier.

9. The pharmaceutical composition according to claim 8, wherein the pharmaceutical composition is in a dosage form of injection, tablet or capsule;

   preferably, the dosage form is selected from solution for injection or lyophilized powder for inj ection;
   preferably, the carrier is selected from excipient, diluent, filler, binder, wetting agent, disintegrant, absorption enhancer, surfactant, absorptive support, and/or stabilizer.

10. A method for treating or preventing inflammatory bowel disease (IBD), comprising administrating to a subject of an effective amount of the fusion protein as defined in any one of claims 1 to 5, or the pharmaceutical composition as defined in claim 8 or 9.

11. The method according to claim 10, wherein the IBD comprise ulcerative colitis, Crohn's disease and indeterminate colitis.

12. The method according to claim 10 or 11, wherein the fusion protein or the pharmaceutical composition is administered orally or by injection.
preferably, the fusion protein or the pharmaceutical composition is administered by subcutaneous or intravenous infusion.

13. The method according to claim 10 or 11, wherein the fusion protein is administered at $3 \times 10^4$ IU to $1 \times 10^6$ IU each dose;
preferably, the pharmaceutical composition is administered at $3 \times 10^4$ IU to $1 \times 10^6$ IU each dose, as measured by the fusion protein therein.

14. The method according to claim 10 or 11, which the fusion protein is administered every 7-28 days, preferably every 14-28 days;
preferably, the pharmaceutical composition is administered every 14-28 days, preferably every 14-28 days.

**FIG. 1**

**FIG. 2**

**FIG. 3**

**FIG. 4**

FIG. 5

FIG. 6

**FIG. 7**

**FIG. 8**

FIG. 9

FIG. 10

**IL-2 Stimulate NK-92 Proliferation**

FIG. 11

**IL-2 Stimulate CTLL-2 Proliferation**

FIG. 12

**SURVIVAL CURVE**

Legend:
- G1: Healthy control group
- G2: Model control group
- G3: $^{125}$Ala IL-2 (30000IU/mouse)
- G4: HSA (0.3 mg/kg)
- G5: IL-2-HSA (10000IU/mouse)
- G6: IL-2-HSA (30000IU/mouse)
- G7: IL-2-HSA (100000IU/mouse)

**FIG. 13**

FIG. 14

FIG. 15

**FIG. 16**

G1: Healthy control group

G2: Model control group

G3: $^{125}$Ala IL-2 (30000IU/mouse)

G4: HSA (0.3 mg / kg)

G5: IL-2-HAS (10000IU/mouse)

G6: IL-2-HAS (30000IU/mouse)

G7: IL-2-HAS (100000IU/mouse)

FIG. 17

**A** Colon length-Day 8  **B** Colon length-Day 15

G1: Healthy control group
G2: Model control group
G3: $^{125}$Ala IL-2 (30000IU/mouse)
G4: HSA (0.3 mg/kg)
G5: IL-2-HSA (10000IU/mouse)
G6: IL-2-HSA (30000IU/mouse)
G7: IL-2-HSA (100000IU/mouse)

**FIG. 18**

A  Colon weight-Day 8

B  Colon weight-Day 15

G1: Healthy control group
G2: Model control group
G3: $^{125}$Ala IL-2 (30000IU/mouse)
G4: HSA (0.3 mg/kg)
G5: IL-2-HSA (10000IU/mouse)
G6: IL-2-HSA (30000IU/mouse)
G7: IL-2-HSA (100000IU/mouse)

FIG. 19

EP 4 394 041 A1

A  Spleen weight-Day 8

B  Spleen organ coefficients-Day 8

C  Spleen weight-Day 15

D  Spleen organ coefficients-Day 15

G1: Healthy control group
G2: Model control group
G3: 125Ala IL-2 (30000IU/mouse)
G4: HSA  (0.3 mg/kg)
G5: IL-2-HSA (10000IU/mouse)
G6: IL-2-HSA (30000IU/mouse)
G7: IL-2-HSA (100000IU/mouse)

FIG. 20

FIG. 21

FIG. 22

**FIG. 23**

Affinity determination between IL-2-HSA and hIL2R

Affinity determination between IL-2-HSA and mIL2R

Affinity determination between IL-2-HSA and rIL2R

Affinity determination between IL-2-HSA and CaIL2R

FIG. 24

EP 4 394 041 A1

Day 3 Comparison of the effects of IL-2-HSA and rhIL-2 on in vitro proliferation human peripheral blood T$_{reg}$ cells

**FIG. 25**

**FIG. 26**

## INTERNATIONAL SEARCH REPORT

| | |
|---|---|
| | International application No. |
| | **PCT/CN2022/106105** |

**A. CLASSIFICATION OF SUBJECT MATTER**

C12N 15/85(2006.01)i; C07K 19/00(2006.01)i; C12N 15/62(2006.01)i; A61K 38/20(2006.01)i; A61K 47/64(2017.01)i; A61P 1/00(2006.01)i

According to International Patent Classification (IPC) or to both national classification and IPC

**B. FIELDS SEARCHED**

Minimum documentation searched (classification system followed by classification symbols)

C12N, C07K, A61K, A61P

Documentation searched other than minimum documentation to the extent that such documents are included in the fields searched

Electronic data base consulted during the international search (name of data base and, where practicable, search terms used)

CNMED, CNABS, DWPI, WPABS, WPABSC, CJFD, AUABS, TWMED, ILABS, HKABS, MOABS, CNTXT, ENTXT, ENTXTC, WOTXT, USTXT, VCN, VEN, GBTXT, EPTXT, CATXT, CNKI, PubMed, EMBL, Web of Science, GoogleScholar: 白细胞介素2, 人血清白蛋白, 炎症性肠病, 溃疡性结肠炎, 克罗恩病, C125A Inflammatory bowel disease(IBD), Interleukin-2(IL-2), Human Serum Albumin (HAS), ulcerative colitis(UC), Crohn's disease, 中国专利生物序列检索系统, Chinese Patent Biological Sequence Retrieval System, NCBI, EMBL: 基于SEQ ID NO: 1, 2, 4的序列检索, Sequence search based on SEQ ID NOs: 1, 2, and 4

**C. DOCUMENTS CONSIDERED TO BE RELEVANT**

| Category* | Citation of document, with indication, where appropriate, of the relevant passages | Relevant to claim No. |
|---|---|---|
| PX | CN 113789346 A (BEIJING VDJBIO CO., LTD.) 14 December 2021 (2021-12-14) claims 1-10 | 1-8 |
| X | CN 113789346 A (BEIJING VDJBIO CO., LTD.) 14 December 2021 (2021-12-14) claims 1-10 | 9 |
| Y | CN 113789346 A (BEIJING VDJBIO CO., LTD.) 14 December 2021 (2021-12-14) claims 1-10 | 10-14 |
| X | CN 112724259 A (SINOBIOTECH (TIANJIN) LTD.; FORTUNEROCK (CHINA) CO., LTD.; TIANJIN SINOBIOTECH LTD.; BEIJING BIO-FORNTUNE LTD.) 30 April 2021 (2021-04-30) claims 1, 5, and 6, sequences 9 and 10, and description, paragraph 160 | 1-9 |
| Y | CN 112724259 A (SINOBIOTECH (TIANJIN) LTD.; FORTUNEROCK (CHINA) CO., LTD.; TIANJIN SINOBIOTECH LTD.; BEIJING BIO-FORNTUNE LTD.) 30 April 2021 (2021-04-30) claims 1, 5, and 6, sequences 9 and 10, and description, paragraph 160 | 10-14 |

☑ Further documents are listed in the continuation of Box C.　　☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **22 September 2022** | **13 October 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/106105** |

**C.    DOCUMENTS CONSIDERED TO BE RELEVANT**

| Category* | Citation of document, with indication, where appropriate, of the relevant passages | Relevant to claim No. |
| --- | --- | --- |
| X | CN 1626554 A (INSTITUTE OF BIOTECHNOLOGY, ACADEMY OF MILITARY MEDICAL SCIENCES, CHINA) 15 June 2005 (2005-06-15)<br>      see abstract | 1-9 |
| Y | CN 1626554 A (INSTITUTE OF BIOTECHNOLOGY, ACADEMY OF MILITARY MEDICAL SCIENCES, CHINA) 15 June 2005 (2005-06-15)<br>      see abstract | 10-14 |
| A | US 2018030106 A1 (INDUSTRY ACADEMY COOPERATION FOUNDATION OF SOONCHUNHYANG UNIVERSITY) 01 February 2018 (2018-02-01)<br>      see abstract | 1-14 |
| Y | 李卓 (LI, Zhuo). "白细胞介素2基因敲除诱发小鼠炎症性肠病的特点及其机制初步分析 (A Preliminary Analysis on the Characterization and Mechanism of Mice IBD Induced by Interleukin-2 Gene Knockout)"<br>生理科学进展 *(Progress in Physiological Sciences)*,<br>Vol. 49, No. 06, 25 December 2018 (2018-12-25),<br>      pp. 459-460 | 10-14 |

Form PCT/ISA/210 (second sheet) (January 2015)

**INTERNATIONAL SEARCH REPORT**

International application No.

**PCT/CN2022/106105**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

    [1] The actually submitted sequence table is an XML file in Standard ST.26.

**INTERNATIONAL SEARCH REPORT**

International application No.

**PCT/CN2022/106105**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **10-14**
   because they relate to subject matter not required to be searched by this Authority, namely:

   [1] Claims 10-14 relate to a method for treating or preventing inflammatory bowel diseases, falling within the scope of methods for treating a human or animal body, and belonging to the cases listed in PCT Rule 39.1(iv) for which no international search is required. Therefore, no international search is conducted. A search was conducted on the basis of a use of the fusion protein according to claims 1-5 or the pharmaceutical composition according to claim 8 or 9 in the preparation of a drug for treating or preventing inflammatory bowel diseases.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2022/106105**

| Patent document cited in search report | | | Publication date (day/month/year) | Patent family member(s) | | | Publication date (day/month/year) |
|---|---|---|---|---|---|---|---|
| CN | 113789346 | A | 14 December 2021 | None | | | |
| CN | 112724259 | A | 30 April 2021 | None | | | |
| CN | 1626554 | A | 15 June 2005 | None | | | |
| US | 2018030106 | A1 | 01 February 2018 | WO | 2016068427 | A1 | 06 May 2016 |
| | | | | WO | 2016068428 | A1 | 06 May 2016 |
| | | | | US | 2018237488 | A1 | 23 August 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022106105 W

**Non-patent literature cited in the description**

- Molecular Cloning: A Laboratory manual. Cold Spring Harbor Laboratory Press